(19) Europäisches Patentamt
European Patent Office
Office européen des brevets

(11) **EP 3 569 717 A2**

(12) **EUROPEAN PATENT APPLICATION**

(43) Date of publication:
**20.11.2019 Bulletin 2019/47**

(51) Int Cl.:
***C12Q 1/686*** *(2018.01)*    ***C12Q 1/70*** *(2006.01)*

(21) Application number: **19194106.1**

(22) Date of filing: **28.08.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Boehringer Ingelheim Vetmedica GmbH**
**55216 Ingelheim am Rhein (DE)**

(72) Inventors:
• **SCHOEDER, Heinz**
  **55216 INGELHEIM AM RHEIN (DE)**
• **HORN, Karolin**
  **55216 INGELHEIM AM RHEIN (DE)**

• **EICKE, Dorothee**
  **55216 INGELHEIM AM RHEIN (DE)**
• **DUCZEK, Juliane**
  **80809 München (DE)**
• **PILL, Nicole**
  **81549 München (DE)**
• **Rebuffo-Scheer, Cecilia**
  **10249 Berlin (DE)**
• **STEHR, Joachim**
  **82178 Puchheim (DE)**
• **BÜRSGENS, Federico**
  **80337 München (DE)**
• **ULLERICH, Lars**
  **80333 München (DE)**

(74) Representative: **Simon, Elke Anna Maria et al**
  **Boehringer Ingelheim GmbH**
  **Binger Strasse 173**
  **55216 Ingelheim am Rhein (DE)**

(54) **DIAGNOSTIC METHOD FOR THE DETECTION OF DNA AND RNA VIRUSES BY CARRYING OUT A POLYMERASE CHAIN REACTION IN A HEATED REACTION VOLUME**

(57)    The present invention is directed to a diagnostic method for the detection of at least one virus selected from African Swine Fever Virus (ASFV) and/or Swine Influenza Virus (SIV) in at least one ex-vivo sample of one or more animals.

EP 3 569 717 A2

SEQUENCE LISTING

<110> Boehringer Ingelheim Vetmedica GmbH

<120> Diagnostic Method for the Detection of DNA and RNA Viruses by carrying out a polymerase chain reaction in a heated reaction volume

<130> 01-3383-EP-1

<160> 18

<170> BiSSAP 1.3.6

<210> 1
<211> 20
<212> DNA
<213> Artificial Sequence

<220>
<223> PCR primer

<400> 1
gataccacaa gatcagccgt                                                    20

<210> 2
<211> 19
<212> DNA
<213> Artificial Sequence

<220>
<223> PCR Primer

<400> 2
ggaggaatac caacccagt                                                     19

<210> 3
<211> 24
<212> DNA
<213> Artificial Sequence

<220>
<223> Hybridisation Probe

<400> 3
ccacgtaatc cgtgtcccaa ctaa                                               24

<210> 4
<211> 26
<212> DNA
<213> Artificial Sequence

<220>
<223> Hybridisation Probe

<400> 4
tgatagaccc cacgtaatcc gtgtcc                                             26

```
<210> 5
<211> 32
<212> DNA
<213> Artificial Sequence


<220>
<223> Hybridisation Probe

<400> 5
taatccgtgt cccaactaat ataaaattct ct                              32


<210> 6
<211> 28
<212> DNA
<213> Artificial Sequence


<220>
<223> Hybridisation Probe

<400> 6
tcatattaac gtatccagag caagagaa                                   28


<210> 7
<211> 20
<212> DNA
<213> Artificial Sequence


<220>
<223> PCR Primer

<400> 7
ctcggtgttg atgaggattt                                            20


<210> 8
<211> 21
<212> DNA
<213> Artificial Sequence


<220>
<223> PCR Primer

<400> 8
accaccacga tgaaaaacta a                                          21


<210> 9
<211> 23
<212> DNA
<213> Artificial Sequence


<220>
<223> Hybridisation Probe
```

<400> 9
tcggagatgt tccaggtagg ttt                                                    23


<210> 10
<211> 20
<212> DNA
<213> Artificial Sequence


<220>
<223> Hybridisation Probe

<400> 10
tcggagatgt tccaggtagg                                                        20


<210> 11
<211> 19
<212> DNA
<213> Artificial Sequence


<220>
<223> PCR Primer

<400> 11
gacgaaaagg caacgaacc                                                         19


<210> 12
<211> 23
<212> DNA
<213> Artificial Sequence


<220>
<223> PCR Primer

<400> 12
gcattgtctc cgaagaaata aga                                                    23


<210> 13
<211> 23
<212> DNA
<213> Artificial Sequence


<220>
<223> Hybrdisation Probe

<400> 13
tactcatgtc aaaggaaggc acg                                                    23


<210> 14
<211> 20
<212> DNA
<213> Artificial Sequence


<220>

<223> Hybridisation Probe

<400> 14
atgtcaaagg aaggcacgat                                                    20

<210> 15
<211> 23
<212> DNA
<213> Artificial Sequence

<220>
<223> PCR Primer

<400> 15
cagaagttat aagaatgatg gaa                                                23

<210> 16
<211> 23
<212> DNA
<213> Artificial Sequence

<220>
<223> PCR Primer

<400> 16
gcattgtctc cgaagaaata aga                                                23

<210> 17
<211> 22
<212> DNA
<213> Artificial Sequence

<220>
<223> Hybridisation Probe

<400> 17
cgatcgggtt cgttgccttt tc                                                 22

<210> 18
<211> 22
<212> DNA
<213> Artificial Sequence

<220>
<223> Hybridisation Probe

<400> 18
gaaaaggcaa cgaacccgat cg                                                 22

**Description**

**FIELD OF THE INVENTION**

The invention relates to the field of diagnostics, in particular to the field of veterinary diagnostics. The invention relates to a diagnostic method for the detection of DNA and RNA viruses by carrying out an amplification of nucleic acids in a reaction volume, which is heated.

**BACKGROUND INFORMATION**

African Swine Fever (ASF) Virus (ASFV) threatens domestic pig populations in Eastern Europe and Asia. While ASFV does not cause disease in humans, the contagious virus causes death in pigs within days. To date, no effective vaccine against this disease exists, therefore the most effective approach to prevent the spread of ASF, are strict animal movement restrictions and slaughtering of affected animals.

Current diagnostic approaches include detection of viral antigens and genomic DNA, as well as detection of antibodies against the virus. These established methods usually take several hours to days from sample-collection to result.

There is a need for the development of rapid diagnostic tests for the detection of ASF. Pulse Controlled Amplification (PCA) technology, a new ultra-fast method for nucleic acid amplification and purification, can support the prevention and spread of infectious diseases such as African swine fewer.

In contrast to conventional PCR (Polymerase Chain Reaction) based platforms, PCA uses microcyclers (gold-coated wires) that are immersed in an amplification solution containing standard PCR components. In PCA one of the primers is covalently attached to the microcycler surface in a functionalization process that allows for the specific binding of target DNA sequences in the solution. Selective heating of microcyclers via an electrical pulse leads to denaturation of nucleic acids and covalently attached primers, which have captured target. The amplification solution itself is held at a constant temperature for annealing and elongation. Selective heating of microcyclers through rapid pulses allows temperature ramps more than 10,000 times faster than in conventional PCR, and therefore allows detection of target DNA within 10 minutes or less.

Another advantage of the microcyclers is their function as nucleic acid extraction elements. Samples such as whole-blood can be added directly to reaction chambers in a hybridization solution which facilitates capture of target molecules (e.g. viral DNA) by primers on the surface of microcyclers. Following target capture, inhibitors can be simply washed out of the chamber, mastermix is added and amplification can be performed without additional purification of the nucleic acids.

US 7,569,366 discloses a method for the amplification of nucleic acids by means of a polymerase chain reaction (PCR), wherein a reaction vessel is arranged on a heating block in order to pre-heat it to an annealing temperature. The reaction vessel is equipped with a heating means in the form of a resistance wire coil or a thin film, which is in direct contact with the reaction volume in the reaction vessel in order to heat this temporarily to an elongation temperature and a denaturation temperature. For this purpose, an electrical current pulse is supplied to the heating means. The pulse duration is to be in the order of at least milliseconds, the denaturation time at least around ten milliseconds. In an exemplary embodiment, the pulse duration is 100 mm (milliseconds) and the volume of the reaction vessel is 200 μl (microlitres).

US 6,586,233 discloses a system for carrying out a PCR, which has a chamber with an upper temperature zone and a lower temperature zone, as well as ducts, which connect the upper and the lower temperature zone to each other. By means of convective pumping, a sample liquid is repeatedly guided through the upper and lower temperature zones in order to achieve a temperature change.

In the international application laid open for public inspection WO 2007/143034, a method is disclosed that is to be suitable for performing a PCR, and with which the temperature change is to be optically induced. It is proposed for example to irradiate nanoparticles with femto-second pulses of a titanium-sapphire laser or nanoparticles or a gold film with an argon-ion laser.

The European application laid open for public inspection EP 2,809,806 discloses a method for the amplification of nucleic acids by means of a PCR on nanoparticles in a reaction volume, which are excited by a laser for the emission of heat. The nanoparticles are conjugated to primers of the PCR.

In the German application laid open for public inspection DE 19543060, a method is disclosed, which is to be suited for carrying out electrochemical measurements on a directly heated, wire-form electrode at temperatures above the boiling point of an electrolyte solution. For this, the sample is heated with short-term, intensive alternating current pulses to high temperatures that lie above the boiling point of the electrolyte solution, while the temperature of the remaining solution is virtually unaffected. Then, the local excess temperature rapidly drops, whereby a boiling of the electrolyte solution and other interference-causing effects are to be avoided. The German application laid open for public inspection DE 199600398 discloses a method which is to be suited for biochemical analysis and wherein an electrode of an electrochemical sensor is modified at its surface with nucleic acid molecules or fractions thereof, which serve as probe sequences for the detection of target sequences. The sensor is heated directly by means of alternating current, wherein merely a very thin layer of a solution under examination close to the electrode surface is heated, but most of the solution remains at virtually unchanged temperature.

Reske, Flechsig et al. report, in "Electrochemical detection of osmium tetroxide- labelled PCR-products by means of protective strands", Talanta 74 (2007), pages 393 to 397, on a method for the electrochemical detection of products of polymerase chain reactions. Here, firstly a conventional PCR is carried out in order to amplify the DNA to be detected. Then, the amplified DNA double strands are separated off and marked with electrochemically active osmium tetroxide bipyridine. These were brought into contact with a gold electrode, on which DNA-probe strands were immobilised. Through voltammetric measurements, the authors seek to have detected a hybridisation of the marked strands to the probe stands.

Duwensee, Flechsig et al. report, in "Electrochemical product detection of an asymmetric convective polymerase chain reaction"; Biosensors and Bioelectronics 25 (2009), pages 400 to 405, on a method for carrying out a PCR by means of convention. For this, a platinum wire was guided through the lower area of a sample tube and was heated to an estimated temperature of 89°C by supplying a heat current for the duration of the experiments of up to 45 minutes. In addition, the sample tube was placed in a water bath with a temperature of 50°C. The authors report that, in the reaction volume, convection with two (eddy) swirls, with central axes running parallel to the heating wire, came about.

WO 2018/073435 describes a method for the amplification of nucleic acids by means of a polymerase chain reaction in a reaction volume, wherein the reaction volume is heated by using electrical energy. In at least one of the passages of the amplification cycle of the polymerase chain reaction, the ratio of the electrical energy used in the denaturation step to heat the reaction volume to the size of the reaction volume is less than 20 Joule per millilitre.

The objective underlying the present invention is therefore to provide a novel diagnostic method for the detection of DNA and RNA viruses, which overcomes the problems of the prior art.

## SUMMARY OF THE INVENTION

In one aspect, the objective of the present invention has surprisingly been solved by providing a diagnostic method for the detection of at least one virus selected from African Swine Fever Virus (ASFV) and/or Swine Influenza Virus (SIV), in at least one ex-vivo sample of one or more animals, comprising the steps:

(a) isolating at least one sample of such one or more animals,
(b) amplifying any present viral genomic nucleic acid contained in such isolated at least one sample by means of a polymerase chain reaction in a reaction volume, wherein the reaction volume is heated through the use of electrical energy, characterised in that in at least one of the passages of the amplification cycle of the polymerase chain reaction, the ratio of the electrical energy used in the denaturation step for heating the reaction volume to the size of the reaction volume is less than 20 Joule per millilitre (mL); and
(c) detecting the presence of the amplified viral genomic nucleic acid contained in such isolated at least one sample of such one or more animals.

In another aspect, the objective of the present invention has surprisingly been solved by providing the diagnostic method as herein described and/or claimed, wherein a heating means consisting of one or a plurality of electrically contacted heating elements, which are in contact with the reaction volume, heats the reaction volume, characterised in that in at least one of the passages of the amplification cycle of the polymerase chain reaction, the heating means supplies to the reaction volume less heat generated in the denaturation step than $C_R * 5°C$, wherein $C_R$ is the heat capacity of the reaction volume during the heating by means of the heating means.

In yet another aspect, the objective of the present invention has surprisingly been solved by providing the diagnostic method as herein described and/or claimed, characterised in that, in at least one of the passages of the amplification cycle of the polymerase chain reaction, the maximum increase of the average temperature of the reaction volume, taking place through the denaturation step, is less than 10°C.

In yet another aspect, the objective of the present invention has surprisingly been solved by providing the diagnostic method as herein described and/or claimed, characterised in that in at least one of the passages of the amplification cycle of the polymerase chain reaction, the heating means supplies to the reaction volume less heat generated in the denaturation step than $C_R * 5°C$, wherein $C_R$ is the heat capacity of the reaction volume during the heating by the heating means and, while the heating means is heating the sample, a temporally stable temperature is not established on at least 10% of the contact area of the heating means with the reaction volume.

In yet another aspect, the objective of the present invention has surprisingly been solved by providing the diagnostic method as herein described and/or claimed, characterised in that the ratio between the surface of the heating element(s), which is in contact with the reaction volume, and the reaction volume is greater than 0.1 per metre.

In yet another aspect, the objective of the present invention has surprisingly been solved by providing the diagnostic method as herein described and/or claimed, characterised in that the heat supply through the heating means varies during the polymerase chain reaction.

In yet another aspect, the objective of the present invention has surprisingly been solved by providing the diagnostic method as herein described and/or claimed, characterised in that in at least one of the passages of the amplification cycle of the polymerase chain reaction, the cycle duration $t_c$ is shorter than 60 seconds.

In yet another aspect, the objective of the present invention has surprisingly been solved by providing the diagnostic method as herein described and/or claimed, characterised in that the duration of the polymerase chain reaction $t_{PCR}$ is shorter than 45 minutes.

In yet another aspect, the objective of the present invention has surprisingly been solved by providing the diagnostic method as herein described and/or claimed, characterised in that the polymerase chain reaction according to step (b) is carried out with the nucleic acid sequences (forward and reverse primers) selected from the group consisting of: SEQ ID NOS: 1, 2, 7, 8, 11, 12, 15, 16, preferably SEQ ID NO: 1 + SEQ ID NO: 2, SEQ ID NO: 7 + SEQ ID NO: 8, SEQ ID NO: 11 + SEQ ID NO: 12 or SEQ ID NO: 15 + SEQ ID NO: 16, wherein more preferably the polymerase chain reaction is a solid-phase polymerase chain reaction, wherein even more preferably one of the nucleic acid sequences (forward and reverse primer) selected from the group consisting of: SEQ ID NO: 1 + SEQ ID NO: 2, SEQ ID NO: 7 + SEQ ID NO: 8, SEQ ID NO: 11 + SEQ ID NO: 12 or SEQ ID NO: 15 + SEQ ID NO: 16, is immobilized on the heating elements as herein described and/or claimed.

In yet another aspect, the objective of the present invention has surprisingly been solved by providing the diagnostic method as herein described and/or claimed, characterised in that such nucleic acid sequences (forward and reverse primers) are directed to ASFV and selected from the group consisting of: SEQ ID NOS: 1,2,7,8.

In yet another aspect, the objective of the present invention has surprisingly been solved by providing the diagnostic method as herein described and/or claimed, characterised in that the nucleic acid sequences (forward and reverse primers) are directed to SIV and selected from the group consisting of: SEQ ID NOS: 11, 12, 15, 16.

In yet another aspect, the objective of the present invention has surprisingly been solved by providing the diagnostic method as herein described and/or claimed, characterised in that the detection according to step (c) is carried out with one or more of the nucleic acid sequences (TaqMan probes) selected from the group consisting of: SEQ ID NOS: 3, 4, 5, 6, 9, 10, 13, 14, 17, 18, preferably wherein such nucleic acid sequences (TaqMan probes) are directed to ASFV and selected from the group consisting of: SEQ ID NOS: 3, 4, 5, 6, 9, 10 or wherein such nucleic acid sequences (TaqMan probes) are directed to SIV and selected from the group consisting of: SEQ ID NOS: 13, 14, 17, 18.

In yet another aspect, the objective of the present invention has surprisingly been solved by providing the diagnostic method as herein described and/or claimed, characterised in that the at least one ex-vivo sample is a blood sample or a tissue sample.

In yet another aspect, the objective of the present invention has surprisingly been solved by providing the diagnostic method as herein described and/or claimed, characterised in that the one or more animals is a mammal, preferably swine, more preferably a domestic pig.

In yet another aspect, the objective of the present invention has surprisingly been solved by providing use of the amplification method as herein described and/or claimed for the detection of at least one virus selected from African Swine Fever Virus (ASFV) and/or Swine Influenza Virus (SIV) in at least one ex-vivo sample of one or more animals.

## DETAILED DESCRIPTION OF THE INVENTION

Before the embodiments of the present invention are described in further details it shall be noted that as used herein and in the appended claims, the singular forms "a", "an", and "the" include plural reference unless the context clearly dictates otherwise.

Unless defined otherwise, all technical and scientific terms used herein have the same meanings as commonly understood by one of ordinary skill in the art to which this invention belongs. All given ranges and values may vary by 1 to 5 % unless indicated otherwise or known otherwise by the person skilled in the art, therefore, the term "about" was usually omitted from the description and claims. Although any methods and materials similar or equivalent to those described herein can be used in the practice or testing of the present invention, the preferred methods, devices, and materials are now described. All publications mentioned herein are incorporated herein by reference for the purpose of describing and disclosing the substances, excipients, carriers, and methodologies as reported in the publications which might be used in connection with the invention. Nothing herein is to be construed as an admission that the invention is not entitled to antedate such disclosure by virtue of prior invention.

In one aspect of the invention, the object thereof is accomplished through a method for the amplification of nucleic acids by means of a PCR in a reaction volume, wherein the reaction volume is heated using electrical energy, wherein, in at least one of the passages of the amplification cycle of the PCR, the ratio of the electrical energy used in the denaturation step to heat the reaction volume to the value of the reaction volume is less than 20 Joule per ml (millilitre).

A PCR in the sense of the present invention is a method for the amplification of nucleic acids, wherein an amplification cycle consisting of the steps of denaturation, hybridisation and elongation is repeatedly passed through, and indeed preferably in this sequence. In each passage of the cycle the number of nucleic acid molecules can be increased (typically doubled in the best case scenario), so that an exponential increase in the number of nucleic acid molecules can arise. A nucleic acid to be amplified is referred to below as an "original". The original is a single strand and can form, together with its complementary strand, which is referred to as a "complement", a double strand. The original and also the complement can be part of a larger nucleic acid. In particular in a PCR, a copy of the original produced in one passage of the amplification cycle can form a template for the formation of a complement in a subsequent passage and a copy of the complement produced can be a template for the formation of an original in a subsequent passage of the cycle. A common term for the amplification product is "amplicon".

The denaturation step serves to denature a nucleic acid double strand, i.e. to separate it into its two single strands. In the denaturation step, for example, the original can be separated from the complement. The type of denaturation that is preferred according to the invention is thermal denaturation (also referred to as "melting"). For this, at least a part of the nucleic acid double strand or the entire double strand is exposed to a temperature, described as the "denaturation temperature", which brings about or at least encourages a separation of the nucleic acid double strands. The preferred denaturation temperature is selected on the one hand to be so high that nucleic acid double strands can be separated. On the other hand the preferred denaturation temperature is selected to be so low that a DNA polymerase, which is possibly also present in the sample, is not significantly damaged. A typical value for the denaturation temperature is 95°C.

To facilitate the following explanation of the invention, "denaturation step" is used in the terminology of the present invention to describe the step of the method, in which the heating means produces heat in order to heat the reaction volume and to bring about a denaturation of double-stranded nucleic acid molecules in this way. The duration of the denaturation step is accordingly the sum of the time, in which the heating means produces heat in the passage of the cycle of the PCR relating to the denaturation step. In the case of a heating resistor being used as a heating means the duration of the denaturation step, thus the duration of a transmission of electricity by the heating means in order to heat the reaction volume and to bring about a denaturation of double-stranded nucleic acid molecules in this way. If the heating means in a passage of the amplification cycle produces the heat not in one but in a plurality of time intervals separated from each other (which can be advantageous, as will be explained below), the duration of the denaturation step is the sum of the durations of these intervals. In the denaturation step defined in this way, in particular the emission of heat based on the heat capacity of the heating means itself is not included and nor is the subsiding of the temperature in the part of the reaction volume adjacent to heating means, even if the temperatures present there are still within the range required for denaturation. This means in particular that, in the method according to the invention, denaturation can also still take place after the thus defined denaturation step. It also means that heat emitted in the denaturation step is generally less than the heat generated in the denaturation step.

Furthermore, the PCR preferably uses at least two oligonucleotides, which are described as "primers": a forward primer and a reverse primer. The forward primer is complementary to the 3'-end of the original and the reverse primer is complementary to the 3'-end of the complement. In the hybridisation step (also referred to as the "annealing step"), the forward primer and/ or the reverse primer hybridise(s) to a sequence complementary thereto in the original or complement or amplicon. The hybridisation step usually takes place at a temperature that brings about or at least encourages a hybridisation of the forward and reverse primers to their complementary sequences in the original or complement or amplicon. It is preferably selected so that it facilitates a hybridisation of the primers that is as specific as possible. The hybridisation temperature is typically between 50°C and 72°C.

In the elongation step, the hybridised primers are complementarily elongated by a polymerase enzyme. Thus, starting from the forward primer, a complement and, starting from the reverse primer, an original can be synthesised. For the purpose of elongation the polymerase is exposed to a temperature that facilitates or at least encourages an elongation. When using a polymerase of Thermus aquaticus (Taq), an elongation temperature of 72°C is typically used. In some embodiments of the PCR the hybridisation and the elongation temperatures are identical, i.e. both steps take place at the same temperature. (This means that there are only two temperature levels during the PCR, a combined hybridisation and elongation temperature and a denaturation temperature.)

The terms "nucleic acid" and "oligonucleotide" include in the context of the present invention not only (desoxy)-ribonucleic acids and (desoxy)-oligoribonucleotides, even if the aforesaid are preferred, but also nucleic acids and oligonucleotides that contain one or more nucleotide analogues with modifications on their backbone (e.g. methylphosphonates, phosphorothioates or peptic nucleic acids (PNA), in particular on a sugar of the backbone (e.g. 2'-O-alkyl derivatives, 3'- and/or 5'-aminoriboses, locked nucleic acids (LNA), hexitol nucleic acids, morpholinos, glycol nucleic acid (GNA), threose nucleic acid (TNA) or tricyclo-DNA - see in this connection the dissertation by D. Renneberg and C.J. Leumann, "Watson-Crick base-pairing properties of Tricyclo-DNA", J. Am. Chem. Soc., 2002, Volume 124, pages 5993-6002, of which the related content is to be regarded as part of the present disclosure by virtue of reference thereto) or that contain base analogues, e.g. 7-deazapurine or universal bases such as nitroindole or modified natural bases such as N4-ethyl- cytosine. In one embodiment of the invention the nucleic acids or oligonucleotides are conjugates or chimera with non-nucleoside analogues, e.g. PNA. In one embodiment of the invention, the nucleic acids or oligonucleotides contain, at one or more positions, non-nucleoside units such as spacers, e.g. hexaethylene glycol or en-spacers with n between 3 and 6. If the nucleic acids or oligonucleotides contain modifications these are selected so that, also with the modification, hybridisation with natural DNA/RNA analytes is possible. Preferred modifications influence the melt behaviour, preferably the melt temperature, in particular in order to be able to differentiate hybrids with different degrees of complementarity of their bases (mismatch discrimination). Preferred modifications include LNA, 8-aza-7- deazapurine, 5-propinyl-uracil and cytosine and/ or abasic interruptions or modifications in the nucleic acid or in the oligonucleotide. Further modifications in the sense of the invention are, e.g., modifications with biotin, thiol and fluorescence donor and fluorescence acceptor molecules.

The method according to the invention takes place in a reaction volume. This means in the sense of the present invention that the amplification of the nucleic acids takes place at least in a part of the cohesive reaction volume. The reaction volume is a liquid solution or suspension, which, besides the solvent or suspension medium, preferably water, usually also contains the nucleic acid(s) to be amplified ("target nucleic acid(s)" is also used below). It generally also contains originals and complements and/ or other constituent parts, for example polymerase(s), dNTPs and salts, which can be suspended or dissolved.

In the sense of the present invention the "electrical energy used for heating" is the energy that is used directly or indirectly to heat the reaction volume. According to this definition it is generally different from the heat supplied to the reaction volume. This difference can hardly be seen or cannot be seen at all for example when using a heating resistor as a heating means, because in this case the electrical energy used for heating is almost completely converted into heat in the heating resistor through the current flow in such a way that with a suitable arrangement of the heating resistor, the electrical energy used for heating can be supplied almost completely to the reaction volume. However, the difference is highly perceivable for example in the case of the method known from EP 2 809 806, wherein nanoparticles are excited with a laser for heat emission, because the electrical energy used for heating in this case is the electrical energy with which the laser is operated, and this does not heat the reaction volume until on the detour via the laser and the nanoparticles. Due to the low degree of efficiency of the detour via the laser and the nanoparticles, only a small part of the electrical energy used for the heating is actually supplied as heat to the reaction volume.

Where reference is made in connection with the present invention to "heating the reaction volume", this does not necessarily mean in the sense of the invention that the whole reaction volume has to be heated, let alone having to be heated evenly. Instead, non-homogeneous heating or heating only of parts of the reaction volume is also regarded as heating of the reaction volume in the sense of this invention.

In a further aspect of the invention the object of the invention is accomplished by a method for the amplification of nucleic acids by means of a PCR in a reaction volume, wherein a heating means consisting of one or a plurality of electrically contacting heating elements, which are in contact with the reaction volume, heats the reaction volume, wherein, in at least one of the passages of the amplification cycle of the PCR, the heating means supplies less heat generated in the denaturation step to the reaction volume than $C_R * 5°C$ (degrees Celsius). $C_R$ is the heat capacity of the reaction volume during the heating by the heating means (both here, and also below, the heat capacity of a body is indicated by the capital letter "C", but the specific heat capacity with by small letter "c"). In other words, without considering other heat inflows and outflows, the heating means heats the reaction volume by on average less than 5°C.

In a further aspect of the invention the object of the invention is accomplished by a method for the amplification of nucleic acids by means of a PCR in a reaction volume, wherein a heating means, which is in contact with the reaction volume, heats the reaction volume, wherein, in at least one of the passages of the amplification cycle of the PCR, the maximum increase of the average temperature taking place through the denaturation step (hereinafter also referred to as "MGTE") of the reaction volume is less than 10°C.

The heating means in the sense of the present invention is thus the sum of the heating elements. In the sense of the present invention "contact" means, with respect to heating elements and reaction volume, that the reaction volume is adjacent to an area of the heating element. If the heating element comprises a material (hereinafter also referred to as a "separating layer"), which is arranged between the heat- generating component of the heating element and the reaction volume, "contact" means that the heating element is in contact, via a face of this separating layer facing towards the reaction volume, with the reaction volume. It is an achievable advantage of this contact between the heating element and reaction volume that the reaction volume can be heated by the heating element in the vicinity of the heating element.

In a further aspect of the invention the object of the invention is achieved by a method for the amplification of nucleic acids by means of a PCR in a reaction volume, wherein a heating means consisting of one or more heating elements, which are in contact with the reaction volume, heats the reaction volume, wherein, in at least one of the passages of the amplification cycle of the PCR, the heating means supplies less heat generated in the denaturation step to the reaction volume than $C_R * 5°C$, and wherein $C_R$ is the heat capacity of the reaction volume during the heating through the heating means, and that no temporally stable temperature gradient is established, during the whole denaturation step, on at least 10% of the contact area of the heating means with the reaction volume.

A temperature gradient is deemed, at a point in time $t_1$ after the start to of the heating by the heating element, to be "temporally stable" in the sense of the present invention if the amount of its maximum incline at a time $t_2 = $ to $+2*(t_1-t_0)$ has changed by less than 20% with respect to the amount of its maximum incline at the point in time t1. In order to ascertain the temporal stability, it is merely the comparison of the amounts of the maximum gradient that is relevant, but not whether or not the heating means generates heat at the point in time $t_2$. Preferably, the amount of the gradient at the point in time $t_2$ has changed by less than 10%, particularly preferably by less than 5%, particularly preferably by less than 3%, particularly preferably by less than 1%. The gradient generally has its maximum incline at the surface of the heating means.

In a further aspect of the invention the object of the invention is achieved by the use of a device comprising a reaction vessel for receiving the reaction volume and a heating means consisting of one or more heating elements, which are in contact with the reaction volume in order to heat it, for the amplification of nucleic acids in a reaction volume, wherein at least one of the heating elements is conjugated to oligonucleotides.

In a further aspect of the invention the object of the invention is achieved by a device for the amplification of nucleic acids in a reaction volume comprising a heating means consisting of one or more heating elements, which can be in contact with the reaction volume, in order to heat it.

In a further aspect of the invention the object of the invention is achieved by a device for the amplification of nucleic acids in a reaction volume, which comprises a heating means consisting of one or more heating elements to heat the reaction volume using electrical energy and a means for bringing the electrical energy into the device, wherein the device is designed so that its electrical power consumption does not exceed 50 W (Watt) at any point in time.

In a further aspect of the invention the object of the invention is achieved by a device for the amplification of nucleic acids in a reaction volume, which comprises a reaction vessel for receiving the reaction volume, a heating means consisting of one or more heating elements to heat the reaction volume using electrical energy and a means for bringing the electrical energy into the device, wherein the device is designed so that the ratio between the electrical power consumption and the capacity of the reaction vessel does not exceed 1 W/ml (Watt per millilitre) at any point in time during the PCR. This restriction does not apply to possibly higher power consumptions during the switch-on process of the device that may be caused by technically based starting currents. Such increased power consumptions are not regarded as power consumptions during the PCR, and are not therefore considered here.

In a further aspect of the invention the object of the invention is achieved by a device for the amplification of nucleic acids in a reaction volume by means of a PCR, which comprises a reaction vessel for receiving the reaction volume, a heating means consisting of one or more heating resistors, and a control device, which applies electrical current to the heating means in order to heat the reaction volume, wherein the control device is designed so that, in at least one of the passages of the amplification cycle of the PCR, the ratio between the electrical energy applied to the heating element by the control device in the denaturation step, and the capacity of the reaction vessel is less than 40 J/ml (Joules per millimetre).

In a further aspect of the invention the object of the invention is achieved by a device for the amplification of nucleic acids in a reaction volume by means of a PCR, which comprises a reaction vessel for the reaction volume, a heating means consisting of at least one heating element, in order to heat the reaction volume, and a control device in order to control the heat emission of the heating means to the reaction volume, wherein the control device is designed so that, in at least one of the passages of the amplification cycle of the PCR, the ratio between the amount of heat emitted by the heating means in the denaturation step to the reaction volume, and the capacity of the reaction vessel for receiving the reaction volume, is less than 20 J/ml, and at least one heating element of the heating means has an expansion of more than $1.5\mu m$ (micrometres) in at least one direction.

The invention is based, inter alia, on the recognition by the inventors that, in known amplification methods, the duration of thermalisation required for the temperatures of the reaction volume that need to be established for the different steps of the nucleic acid amplification makes a considerable contribution to the method duration and that the method duration could be shortened by shortening these phases. It is further based on the recognition by the inventors that the method can also be carried out efficiently when one or more temperature(s) required for the PCR are only achieved in a part of the reaction volume. The invention is also based on the recognition that this can be achieved with significantly lower energy use than in the prior art.

In particular it can be achieved with the invention that - for example with the aid of short electrical impulses - only the direct vicinity of the heating element(s) of the heating means is heated for a short time, preferably in order to carry out the denaturation of the nucleic acid molecules in the reaction volume, while the majority of the reaction volume remains at a (in this sense "global") base temperature, at which in particular an elongation, preferably also a hybridisation, can take place. This is preferably achieved by the duration of the heating through the heating means being so short that the thermal field arising in the surrounding reaction volume can only spread a few micrometres and in this way creates a heating-up zone, which preferably comprises only a tiny fraction of the reaction volume. In particular, the amount of heat brought can be so low that no substantial global heating of the reaction volume takes place.

The "global temperature" in the sense of the present invention is the average temperature, with respect to volume, of the reaction volume, thus the temperature that is established or would be established in the reaction volume after a thermalisation thereof. The "global heating" is the increase in the global temperature defined in this way.

Furthermore it can be achieved with the invention that, after heating, in particular in the denaturation step, the heat brought, which spreads from the heating-up zone into the rest of the reaction volume, only brings about a negligible global temperature increase there. "Negligible" means here in particular that the temperature increase is preferably too low for a denaturation of the nucleic acid molecules and particularly preferably that the temperature increase is too low to interfere with the hybridisation and the elongation.

The denaturation and preferably also other steps of the nucleic acid amplification can thus take place locally in the direct vicinity of the heating elements, wherein at least one of the required primers is fixed (hereinafter referred to as: "functionalised") on the heating means, in order to allow the amplicon also to form there and thus to facilitate a denaturation with local heating. In other words, due to the fact that, based on the functionalisation of the heating means, a localisation of steps of the PCR, in particular hybridisation, elongation and/ or denaturation, as well as preferably also the generation of a signal to observe the progress of the PCR, is achieved in the direct vicinity of the heating means, the heating of the reaction volume can be limited to a fraction of the reaction volume.

It is achievable with the invention that nucleic acids can be amplified more rapidly. In particular, in contrast with conventional thermocyclers, in which the heating and cooling processes last many seconds, it can be achieved with the invention that the duration of the PCR is no longer determined by technical limitations such as the heating and cooling rates. Thermalisation times in the reaction vessel can also be omitted, as the heat is constantly generated in the vicinity of the heating means. The inventors ascertained that, even in the case of 40 passages of the amplification cycle of a PCR, the denaturation of the nucleic acid molecules and the cooling thereof to an elongation and hybridisation temperature, only take a few milliseconds in total. It can be achieved through the invention that the duration of the PCR is determined predominantly by the durations between the denaturation steps that are required for the diffusion and reaction processes and the biochemical processes such as elongation by the polymerase.

It is also achievable with the invention that nucleic acids can be amplified with lower energy use. Furthermore the invention enables the amplification process to be controlled more effectively and for example to extensively avoid the temperature fluctuations of the reaction volume that arise within the temperature control in the known methods. More cost-effective and more compact device for the amplification of nucleic acids can be provided by the invention. For example it can be achieved to provide a device according to the invention for the amplification of nucleic acids in the form of a universal serial bus (USB) stick.

Advantageous embodiments and refinements, which can be used individually or in combination with each other, are the subject matter of the dependent claims.

In a preferred embodiment of the invention, in at least one, preferably in at least three, particularly preferably in at least 10, particularly preferably in at least 20, of the passages of the amplification cycle of the PCR, the ratio of the electrical energy used in the denaturation step for heating the reaction volume to the size of the reaction volume is less than 20 J/ml, preferably less than 10 J/ml. With this embodiment of the invention a low energy consumption of the method according to the invention can advantageously be achieved. In addition it the addition of energy in the denaturation step being so great that it leads to an excessive global heating of the reaction volume can be advantageously avoided.

In a preferred embodiment of the invention, in at least one, preferably in at least three, particularly preferably in at least 10, particularly preferably in at least 20, of the passages of the amplification cycle of the PCR, the ratio of the electrical energy used in the denaturation step for heating the reaction volume to the size of the reaction volume is between 0.01 and 30 W/ml (Watt per millilitre), preferably between 0.05 and 10 W/ml, and particularly preferably between 0.1 and 5 W/ml.

In one embodiment of the invention the denaturation step comprises a plurality of time intervals spaced apart from each other, in which the heating means produces heat in the passage of the cycle of the PCR relating to the denaturation step. In each case the time-based distance of the time intervals of the current pulses must be very much smaller than the sample thermalisation time; particularly preferably the time- based distance of the time intervals of the current pulses must be selected so that the temperature of the heating means between the time intervals decreases less than 20%, This embodiment of the invention can be favorable for example if a switching power supply is used for energy supply. Particularly preferably, the denaturation step is realized through a current impulse or a plurality of current impulses, wherein the absolute amount of the current strength during the time duration or the current impulse(s) in a preferred embodiment varies by less than 10%. In this way, smooth dynamics of the heating of the reaction volume can be achieved.

In a preferred embodiment of the invention in at least one, preferably in at least three, particularly preferably in at least 10, particularly preferably in at least 20, of the passages of the amplification cycle of the PCR, the ratio of the electrical energy used in the denaturation step for heating the reaction volume to the size of the reaction volume is greater than 10 J/ml (millijoules per millilitre), preferably greater than 30 J/ml and particularly preferably greater than 100 mJ/mL. The energy is calculated in one embodiment with constant electrical power used simply from the product of this used electrical power and the duration of the denaturation step. It is an achievable advantage of this embodiment of the invention that the energy is sufficiently great In order to operate a heating means with an area in contact with the reaction volume, of which the size is sufficient in order to meet the requirements that are common in practice for the reaction kinetics of the PCR.

Preferably in at least one, particularly preferably in at least three, particularly preferably in at least 10, particularly preferably in at least 20, of the passages of the amplification cycle of the PCR, the heating means supplies to the reaction volume less heat generated in the denaturation step than $C_R$ * 5°C (Celsius). $C_R$ is hereby the heat capacity of the reaction volume during the heating by the heating means. In other words, if other heat inflows and outflows are left out of consideration, the heating means heats the reaction volume by on average less than 5°C.

Preferably in at least tone, particularly preferably in at least three, particularly preferably in at least 10, particularly preferably in at least 20, of the passages of the amplification cycle of the PCR, the maximum MGTE of the reaction volume is less than 10°C, particularly preferably less than 7°C, particularly preferably less than 5°C, particularly preferably less than 4°C, particularly preferably less than 3°C, particularly preferably less than 2°C, particularly preferably less than 1°C, particularly preferably less than 0.75°C, particularly preferably less than 0.5°C and most particularly preferably less than 0.3°C.

Values for the MGTE of 4°C and more can be advantageous in particular if the combined hybridisation and elongation temperature increases in the course of the PCR (for example because the local amplicon density on the heating element greatly increases in the course of the PCR), i.e. in order to also (slightly) change the global temperature via the local heating. In addition, in this way the heat power provided externally, for example by a temperature-regulating block can be reduced, which can further reduce the total power requirement of a device according to the invention. Low values for the MGTE can be advantageous in order to be able to carry out passages of the amplification cycle of the PCR one after the other in very quick succession, or, in order to be able to thermally insulate the sample well with respect to the vicinity and thus make it independent of ambient conditions. If for example the combined hybridisation and elongation temperature is 70°C and the volumetrically averaged temperature of the reaction volumes after a denaturation step increases to 70.6°C, then the MGTE = 0.6°C here.

In a preferred embodiment of the invention, in at least one, preferably in at least three, particularly preferably in at least 10, particularly preferably in at least 20, of the passages of the amplification cycle of the PCR, during the denaturation step no temporally stable temperature gradient is established on at least 10%, preferably at least 30%, particularly preferably at least 50%, particularly preferably at least 80%, of the contact surface of the heating means with the reaction volume.

In a preferred embodiment of the invention in at least one, preferably in at least three, particularly preferably in at least 10, particularly preferably in at least 20 of the passages of the amplification cycle of the PCR, for at least half the duration of the denaturation step, the maximum incline of the temperature gradient is greater than 0.5°C/$\mu$m, particularly preferably greater than 1°C/$\mu$m, and most particularly preferably greater than 3°C/ $\mu$m. An achievable advantage of this embodiment of the invention is achieving a good localisation of the temperature increase brought about by the heating element, as a localisation of the temperature increase necessarily requires the formation of a gradient (the temperature gradient does not therefore have to be temporally constant in this embodiment).

In a preferred embodiment of the invention in at least one, preferably in at least three In a preferred embodiment of the invention in at least one, preferably in at least three, particularly preferably in at least 10, particularly preferably in at least 20 of the passages of the amplification cycle of the PCR, for at least half the duration of the denaturation step, the maximum incline of the temperature gradient is less than 1000°C/$\mu$m, particularly preferably less than 300°C/$\mu$m. It is an achievable advantage of this embodiment of the invention that thermophoretic effects in the solution can be avoided.

In a preferred embodiment of the invention in at least one, preferably in at least three, particularly preferably in at least 10, particularly preferably in at least 20 of the passages of the amplification cycle of the PCR, the cycle duration $t_c$ is shorter than 60 s (seconds), preferably shorter than 40 s, particularly preferably shorter than 20 s, particularly preferably shorter than 15 s, particularly preferably shorter than 10 s. In the sense of the present invention the cycle duration $t_c$ is the duration of a passage of the amplification cycle of the polymerase chain reaction consisting of the steps of denaturation, hybridisation and elongation in this sequence. By selecting a particularly short cycle duration $t_c$, a particularly rapid PCR method can be realised in this embodiment of the invention.

In a preferred embodiment of the invention in at least one, preferably in at least three, particularly preferably in at least 10, particularly preferably in at least 20 of the passages of the amplification cycle of the PCR, the duration of the PCR $t_{PCR}$ is shorter than 45 minutes, particularly preferably shorter than 30 minutes, particularly preferably shorter than 20 minutes, particularly preferably shorter than 15 minutes and particularly preferably shorter than 10 minutes. The duration is the time from the start to the end of the PCR, wherein the start of the PCR is the point in time of the start of the denaturation step of the first complete passage of an amplification cycle consisting of the steps of denaturation, hybridisation and elongation in this sequence, and the time of the end of the PCR is the end of the denaturation step of the last complete passage of the amplification cycle with the steps of denaturation, hybridisation and elongation in this sequence.

Heating means

In a preferred embodiment of the invention the reaction volume is heated by a heating means made up of one or more heating elements, wherein particularly preferably the heating element or at least one of the heating elements, particularly preferably all heating elements, are in electrical contact. It can advantageously be achieved in this way that current can flow through the heating element. This embodiment of the invention can utilize the fact that electrically contact heating elements can be operated and regulated particularly simply by an electrical control device.

The heating element, or one of the heating elements, is preferably a device that converts a current flow into heat, for example through its ohmic resistance. This embodiment of the invention can exploit the fact that such heating elements can convert electrical energy efficiently into heat. A preferred heating element is a heating resistor or a Peltier element. In the case of a plurality of heating elements, these can be arranged in series or in parallel, or partially in series and partially in parallel.

Preferred heating elements can have a material, which is arranged between the heat-generating component of the heating element and the reaction volume. Such a material can be useful in order to protect the heating element for example from corrosion or other chemical interactions with the PCR chemistry and/or to electrically insulate it (it is thus described here as a "separating layer") and can consist for example of polymers. Preferably such heating elements and the reaction volume are maximally separated by a separating layer of a thickness of less than 500 $\mu$m, preferably a thickness of less than 100 $\mu$m, preferably a thickness of less than 20 $\mu$m, preferably a thickness of less than 5 $\mu$m, preferably a thickness of less than 1 $\mu$m, preferably a thickness of less than 0.2 $\mu$m, preferably a thickness of less than 0.05 $\mu$m. Most particularly preferable is a further embodiment, wherein no separating layer is present between heating elements and reaction volume, or wherein the thermic properties of the separating layer are selected so that their effect with regard to the heat emission of the heating means to the reaction volume is negligible.

It can be advantageous for an even, or steady, temperature to be reached as far as possible everywhere on the heating elements. This can be achieved in the case of heating resistors for example in that, in each sub-piece or sub-volume of the heating resistor, an even current density during the heating and a constant surface/ volume ratio (OVV) are facilitated. This is advantageously achievable in that a constant conductor cross-section and a constant voltage drop per unit of length of the conductor are ensured in the whole heating element. In the case of a plurality of heating elements, these heat the reaction volume in a preferred embodiment of the invention in the same way. However, the invention also includes embodiments in which heating elements heat the reaction volume differently, for example for different lengths of time or with different intensities. The heating elements of the heating means can be the same or different, for example with regard to their length or geometry.

The reaction volume is preferably heated by a heating means made up of one or more heating elements, wherein particularly preferably the heating element or at least one of the heating elements, particularly preferably all the heating elements, are in contact with the reaction volume. In a preferred embodiment of the invention at least one of the heating elements abuts, on its entire surface, against the reaction volume. Particularly preferably all the heating elements abut against the reaction volume on their entire surface. It is an achievable advantage of this embodiment of the invention that the reaction volume can be efficiently heated by the respective heating element in the vicinity of the heating element. It can also be advantageously achieved through this embodiment of the invention that, in order to ensure high reaction kinetics (especially of the hybridisation kinetics of the nucleic acid molecules to primers, which, as indicated below, are preferably arranged on the heating element), the heating means has an accessible surface that is as large as possible.

It is preferable for the heating elements to have a surface/ volume ratio (OVV) that is as high as possible in order to facilitate an emission of the heat that is as effective as possible to the (direct) vicinity, and at the same time to have a volume that is as low as possible, in order to ensure a low heat capacity of the heating element. Preferred embodiments according to the invention have a surface/ volume ratio for the heating elements that is more than $10^3$ m$^{-1}$ (per metre), preferably more than $10^4$ m$^{-1}$ and particularly preferably more than $5 * 10^4$ m$^{-1}$. A surface/ volume ratio that is too great, however, can lead in some cases to very filigree and thus mechanically unstable structures, so that it can be advantageous according to the invention to keep the surface/ volume ratio less than $10^9$ m$^{-1}$, preferably less than $10^8$ m$^{-1}$ and in some cases even less than $10^7$ m$^{-1}$.

For a long wire draht (length much greater than diameter) the surface/ volume ratio is calculated for example with 2/r, wherein r is the radius of the wire. For a thin film or a foil (thickness very much less than length and lateral expansion), the surface/ volume ratio is calculated with 1/d, wherein d is the thickness of the film or the foil. According to the invention it is preferable, for the above embodiments, to take into consideration only the surface that is in contact with the reaction volume. It is also preferable to take into consideration only the volume of which surface(s) is/ are in contact with the reaction volume (i.e., for example, inlet lines that do not run through the solution are not to be regarded according to the invention as relevant volumes and surfaces). The same also applies correspondingly to the subsequent consideration of volume fill factor and heat capacity.

In a preferred embodiment of the invention the ratio between the surface of the heating means that is in contact with the reaction volume and the reaction volume is greater than 0.1 m$^{-1}$, particularly preferably greater than 1 m$^{-1}$,

particularly preferably greater than 5 m$^{-1}$, particularly preferably greater than 10 m$^{-1}$, particularly preferably greater than 20 m$^{-1}$, particularly preferably greater than 50 m$^{-1}$, particularly preferably greater than 100 m$^{-1}$. This embodiment of the invention advantageously enables favorable reaction kinetics to be achieved in that, in a large proportion of the reaction volume, constituent parts of the reaction volume can rapidly reach the surface of the heating element through diffusion, in order to participate in the steps of the nucleic acid amplification method taking place there. Also, in the case described further below for heating elements, which are functionalized at least in part on their surface with one of the reaction partners (for example a primer), it is possible to utilize the fact that more reaction partners are also available through a larger surface.

In order to prevent the heating element structure becoming too filigree or the movement of the nucleic acid molecules and other reactants located in the reaction volume being hindered by too many surfaces, the ratio of the surface of the heating element or the heating elements in the ratio to the size of the reaction volume is less than 10$^6$ m$^{-1}$, particularly preferably less than 10$^5$ m$^{-1}$, particularly preferably less than 10$^4$ m$^{-1}$, and most particularly preferably less than 10$^3$ m$^{-1}$.

In order to keep the heat supplied by the heating means to the reaction volume in the denaturation step as low as possible, it can be advantageous to also keep the heat capacity of the heating means low, as, in order to achieve a certain temperature increase on the surface of the heating elements: an increasingly large amount of energy is required, the greater the heat capacity of the heating means. The amount of energy supplied to the reaction volume by the heating means in the denaturation step subsequently spreads over the whole reaction volume. The heat capacity of the heating element is given by the product of the respective volume and the specific volumetric heat capacity of the respective material, from which the respective volume is made. A significant degree of freedom in the configuration of the heating means is in its dimensions.

It can therefore be advantageous to keep the volume of the heating means, in particular the material thickness, as low as possible. It is noteworthy in this respect that the heat diffusion range does not depend on the size of the heating means but instead merely on the heating duration. In a preferred embodiment of the invention the volume of all the heating elements of the heating means is less than 10%, preferably less than 5%, particularly preferably less than 3% and most particularly preferably less than 1% of the reaction volume. With this embodiment of the invention, a low heat capacity of the heating means can advantageously be achieved through a low volume fill factor.

In a preferred embodiment of the invention the heating means comprises an arrangement of a plurality of conductors with current flowing through them, which are surrounded by the reaction volume. It is can advantageously be achieved here that the heating means can be effective at many different places, or parts, of the reaction volume. This is particularly favorable in typical cases, in which at the start of a PCR there is only a very low concentration of nucleic acid molecules to be amplified and the average distance of these molecules from the nearest heating element is therefore long. It can be estimated that a nucleic acid single strand with a length of 100 base pairs requires a time $t = x^2/D_{DNA}$ in order to move itself through diffusion, over a distance x from its starting point (wherein $D_{DNA} \approx 10^{-11}$ m$^2$/s). In a preferred embodiment of the invention the spatial distance of a given point in the reaction volume from the nearest heating element is less than 3 mm (millimetres), preferably less than 2 mm, particularly preferably less than 1 mm, particularly preferably less than 0.75 mm, particularly preferably less than 0.5 mm and particularly preferably less than 0.25 mm

In an embodiment of the invention one or all of the heating elements is/ are made from an electrically conductive metal or a metal alloy or another electrically conductive material with low specific electrical resistance for example carbon, a semiconductor material or a conductive plastic.

In one embodiment, one or all of the heating elements consist(s) only of one wire or one electrical conductor or a plurality of wires or electrical conductors. These can be straight, bent or wound in a coil. A plurality of wires or electrical conductors can have an equal or unequal distance from each other, can cross each other or not cross each other. Each wire or electrical conductor can have a round, oval, planar or any other cross-section. A heating element can also be simply a passable, i.e. a pass- though, surface area, for example a flat surface damped with metal.

A particularly preferred embodiment of the heating means is an arrangement, or array, preferably a periodic arrangement, of conductive metal wires. Particularly preferably, the wires are arranged parallel to each other. The wires of the array preferably have a diameter of between 0.5 and 100 $\mu$m (micrometers), particularly preferably between 1 and 50 $\mu$m. The wires are preferably spaced apart from each other by between 50 and 1500 $\mu$m.

The wires preferably are made of gold or other metals, or they are designed as sheathed wires, wherein the core consists of a cheaper and more stable material, preferably a metal. Particularly preferable are sheathed wires with stainless steel, molybdenum or most particularly preferably with a tungsten core with a sheath of an inert material, preferably gold. Due to the high strength of the core, such wires can advantageously be designed to be very thin (and thus with a high surface/ volume ratio), but still facilitate, through the sheath material, still the desired chemical properties of the preferably stainless steel sheath. Particularly preferred are sheathed wires with a tungsten core (tungsten advantageously has a much higher tensile strength than gold), preferably with a core diameter of between 5 and 40 $\mu$m and a gold sheath with a thickness of between 0.1 and 2 $\mu$m.

In another embodiment metallic foils are used as the heating element, which traverse the reaction volume and which designed as lattices, for example through stamping/ punching, electroforming, laser or hydro-jet cutting, etching technology or other methods.

In another embodiment of the invention the heating element is applied to a material that is not electrically conductive or which has poor electrical conductivity. Preferred heating elements are made of a metallic film, which has been applied to a non- conductive structure galvanically, chemically, through PVD, through pressure methods or other methods. The non-conductive structure can for example be designed as a very fine injection molded part (a preferred structure size is less than 300 $\mu$m) or through a rapid prototyping method. Fabric structures can also be considered for use as heating elements or carriers for heating elements. In particular such materials with a mesh size of between 20 $\mu$m and 3 mm, particularly preferably between 100 $\mu$m and 1.5 mm, can be used as heating elements or as carriers for heating elements. If the fabric structures themselves are conductive, they can advantageously be used in their entirety as a heating element. If they are made of non-conductive material (for example a plastic), they can be metal-plated, so that the current flows only through a thin surface layer (typically< 10 $\mu$m) and so that there is a large surface. In other words, the wires or fibers of the fabric of lattice have a comparatively large surface, but only the thin, applied metallic volume is actively heated. In the sense of the invention it is only the part through which the current essentially flows that is to be regarded as the heating element. If for example a plastic structure of PMMA with a gold film is evaporated, then only the gold film to be regarded as a heating element.

Functionalization of the heating means

In a preferred embodiment of the invention at least one of the heating elements of the heating means is conjugated to oligonucleotides, i.e. oligonucleotides are joined to the heating element. Particularly preferably all the heating elements of the heating means are conjugated to oligonucleotides. In this way it can advantageously be achieved that oligonucleotides that are parts of the method according to the invention are specifically heated by the heating means without the whole reaction volume having to be heated. In a particularly preferred embodiment of the invention the heating element(s) is/ are conjugated to primers, most particularly preferably to forward and reverse primers of the PCR method. In a preferred embodiment of the invention forward primers, but no reverse primer, are attached to a heating element or a portion of a heating element, and / or reverse primers, but no forward primers, are attached to a heating element or a portion of a heating element. The molecules of the other primer, in each case, can be freely suspended in the reaction volume. If a separating layer is used between heat generating components of the heating means and the reaction volume, the functionalization must be realized in such a way that oligonucleotides are accessible from the liquid volume, i.e. they are preferably attached on the surface of the separating layer.

In a further preferred embodiment, at least one of the heating elements is conjugated to forward primers and also to reverse primers. In a particularly preferred embodiment all heating elements of the heating means are conjugated to forward primers and also to reverse primers. With this embodiment of the invention, it can advantageously be achieved that the PCR product of a forward primer, for hybridization with the reverse primer of the same heating element, only needs to travel a short distance, with the result that a hybridization can take place more quickly and therefore the PCR method can be carried out more quickly.

In a preferred embodiment of the invention a heating element is provided at its surface with a material that allows the bonding of nucleic acids. For example a gold-plated surface can be used in order to bind a primer via one or more thiol bond(s) on a heating element. Also, for example a streptavidin biotin bond can be used to bind a primer to the heating elements if, for example, preferably beforehand, one of the two partners (streptavidin or biotin) has been bonded to the heating elements and the primer (at the 5'-end) is modified with the other of the two partners and subsequently thereby bonded to the heating element. Other modifications such as, for example, amino or carboxy groups, can also be used to bind primers to the heating elements; for this purpose, the surface of the heating element can for example, preferably beforehand, be modified with epoxy. A bond is preferably realized in such a way that the 5'-end of the primer is bonded to the heating elements, so that the 3'-end is free and can therefore be elongated during the PCR by the polymerase.

In the embodiment in which both forward primers and also reverse primers are immobilized on a heating element, the distance between the primer molecules of different types can purposefully be selected to that on average they are at a distance from each other of, for example, less than 1 nm (nanometer), less than 3 nm, less than 5 nm, less than 15 nm or less than 50 nm. With this embodiment, it can advantageously be utilized that, as soon as a forward primer has been elongated on the surface of the heating element, this newly written nucleic acid strand hybridizes, after denaturation, to a corresponding adjacent reverse primer molecule on the surface or to a reverse primer molecule in the vicinity. As this process takes place on the surface of the heating elements, the local concentrations are extremely high. The new nucleic acid strand does not therefore have to travel many micrometers through diffusion in order to find a reverse primer molecule, because in the direct vicinity - only a few nanometers away - there are many reaction partners (the same applies, vice versa, to elongated reverse primer molecules, which find immobilized forward primer molecules in the vicinity).

In one embodiment, besides the primer molecule(s), there are also fill oligonucleotides or fill molecules on the heating elements, i.e. oligonucleotides or molecules that do not actively participate as primers or fluorescence probe or target nucleic acid in the PCR, but instead merely serve for surface saturation or passivation, in order to prevent a nonspecific (thus not through targeted hybridization with primers) binding of am amplicon or a target nucleic acid to the surface of the heating element. Fill oligonucleotides preferably have a length of between 5 and 50 nucleotides, particularly preferably between 10 and 40 nucleotides and most particularly preferably between 20 and 30 nucleotides. In particular, they can consist only of a nucleotide type, for example A30 sequences with 30 adenine bases. Fill molecules can for example be biotin or polyethylene glycol, for example additionally provided with a functional group such as for example thiol, in order to immobilize the fill molecules on the heating element surface. However, fill molecules can also be for example bovine serum albumen.

In a further preferred embodiment of the method the oligonucleotides on the heating elements have a spacer sequence as a sub-sequence. The spacer sequence is thereby on the side, facing towards the heating, of the respective oligonucleotide. The spacer sequence thus serves as a spacer for the rest of the oligonucleotide. In a preferred embodiment an oligonucleotide contains both a sub-sequence, which has the function of a primer and is described as a primer sequence, and also a sub- sequence that is a spacer sequence. Due to the fact that the primer sequences are spaced further apart from the heating elements by the spacer sequences, the nucleic acids to be amplified and the DNA polymerases advantageously have better access to the primer sequences.

In a preferred embodiment of the method according to the invention, there is one or more non-basic (abasic) modification(s) between spacer sequence and primer sequence, these abasic modifications preventing the overwriting of the spacer sequence by the polymerase. The content of the patent application DE 102013215168 in this respect is to be regarded as part of the present disclosure by virtue of this reference thereto. Such modifications can be for example: 1',2'-dideoxyribose (dSpacer), triethylene glycol spacer (spacer 9) or hexaethylene glycol spacer (spacer 18), which prevent the further polymerase activity in 3'-direction. In this way it can be achieved that the spacer sequence does not serve as a template for the nucleic acid strand synthesized by the polymerase and the resulting PCR product does not become unnecessarily long. An elongated PCR product, which also contains the complementary sequence to the spacer sequence, would have a significantly increased melt temperature with the oligonucleotides on the nanoparticles and would hybridize unnecessarily non-specifically in subsequent hybridization steps and thus make the whole PCR more nonspecific.

Duration of the heating

The heat supply through the heating means preferably varies during the PCR. Particularly preferably, the heat supply through the heating means varies during at least one passage of the amplification cycle of the PCR, particularly preferably during at least three, particularly preferably in at least 10, particularly preferably in at least 20, of the passages of the amplification cycle of the PCR, particularly preferably periodically.

The duration of the heating by the heating means in the denaturation step (hereinafter the heating by the heating means in the denaturation step is also referred to as "heat pulse") in a preferred embodiment of the invention is, in at least one, preferably in at least three, particularly preferably in at least 10, particularly preferably at least 20 of the passages of the amplification cycle of the PCR, is in the interval between 100 ns (nanoseconds) and 30 ms, particularly preferably between 0.5 $\mu$s (microseconds) and 10 ms, particularly preferably in the interval between 1 $\mu$s and 5 ms, particularly preferably between 1 $\mu$s and 3 ms, particularly preferably between 1 $\mu$s and 1 ms, particularly preferably between 1 $\mu$s and 800 $\mu$s, and most particularly preferably in the interval between 1 $\mu$s and 500 $\mu$s. An achievable advantage of this embodiment of the invention is a localization of the heat and thus the resulting temperature distribution. In other words, due to the short heating duration, limited heat is transported by the heating element through heat diffusion into the solution. At the same time it can be achieved with this embodiment of the invention that the heating duration is not too short to allow a sufficient melting or extrication of the nucleic acid double strands during the time of the local heating, and I or to enable, during the time of the local heating, both single strands to be able to move sufficiently far away from each other through Brownian motion (and/ or other forces) so that they do not re-hybridize to each other.

In a preferred embodiment of the invention the duration of the denaturation step accounts for only a small fraction of the total duration of the PCR. Preferably the denaturation step accounts, during at least one passage of the amplification cycle, particularly preferably during at least three, particularly preferably during at least 10, particularly preferably during at least 20 of the passages of the amplification cycle of the PCR, less than 10%, furthermore preferably less than 5%, particularly preferably less than 3%, particularly preferably less than 1%, particularly preferably less than 0.5%, particularly preferably less than 0.05%, and particularly preferably less than 0.01% of the time taken by the entire passage of the amplification cycle of the PCR. Through this embodiment of the invention it can advantageously be achieved that hybridizations can take place during virtually the entire duration of the PCR. As the polymerase in the local PCR can work for virtually the entire duration, the process time can be shortened. It is also achievable that, due to the heating being realized only locally realized and in addition very short heating, the participating polymerase enzymes and also other reaction partners are protected and they lose their processivity less quickly.

In a preferred embodiment of the invention, during at least one passage of the amplification cycle, particularly preferably during at least three, particularly preferably during at least 10, particularly preferably during at least 20 of the passages of the amplification cycle of the PCR, the duration of the denaturation step $t_{heat}$ is shorter than $t_{heat} \leq (s1 \cdot |x|)^2/D$, wherein s1 is a scaling factor, |x| is the critical distance and D is the temperature conductivity. The scaling factor s1 is preferably s1=100, particularly preferably s1=10, particularly preferably s1=1, particularly preferably s1=0.1, particularly preferably s1=0.01. The critical distance |x| is the distance from the nearest indirectly adjacent part of the heating means, for example from the nearest heating element of the heating means. If the heating element(s) is/ are constructed from a 2D structure (for example a lattice, fabric, honeycombs, etc.), the mesh size or the size of the holes / recesses is the relevant value |x|. If the heating element(s) consist(s) of a 3D structure, the pore size is the relevant value |x|.

It can be achieved with this embodiment of the invention that the heating duration is so short that the heat diffusion range is much smaller than the average distance |x|, thermal fields of adjacent heating elements or generally adjacent, non-abutting parts of the heating means do not therefore overlap. In particular, scaling factors greater than 1 can be advantageous for very long amplicons, wherein the disentangling of the two nucleic acid strands takes longer (the time taken until a nucleic acid double strand can disentangle through Brownian motion) increases to the fourth power of the length. Scaling factors below 1 can be advantageous for the best possible heating and cooling dynamics.

Electricity storages

A preferred device according to the invention is configured so that its electrical power consumption does not at any time during the PCR exceed 50 W, particularly preferably 20 W, particularly preferably 10 W, particularly preferably 3 W, particularly preferably 2.5 W, particularly preferably 1.5 W, particularly preferably 0.5 W. This limitation does not apply to possibly higher power consumption during the switch-on process of the device, as may be caused for technical reasons by switch-on currents. Such increased power consumptions are not regarded as power consumptions during the PCR and are not therefore taken into consideration here. With this embodiment of the invention it can advantageously be achieved that the device can be operated on common portable power sources, for example on a motor vehicle battery, on the cigarette lighter of a motor vehicle or on a port of a PC, a tablet computer or a mobile phone, for example on an USB or an Apple lightning connection.

The device preferably comprises an electricity storage. It is preferably designed so that the electrical energy retained in the electricity storage, in relation to the capacity of the reaction vessel, is greater than 0.1 J/ml, particularly preferably greater than 1 J/ml, particularly preferably greater than 2 J/ml, particularly preferably greater than 3 J/ml. The electricity for the electricity requirements of the heating means, which also varies due to varying heat supply, can be intermediately stored with the electricity storage.

A device according to the invention comprising an electricity storage is preferably designed so that the electrical energy retained in the electricity storage energy, in relation to the capacity of the reaction vessel, is less greater than 100 J/ml, particularly preferably less than 50 J/ml, particularly preferably less than 30 J/ml. This embodiment of the invention can utilise the fact that, due to particularly efficient heating through the heating means, the electricity storage can have a small configuration. In this way the device according to the invention can advantageously be designed to be particularly compact, cost-effective and portable.

A preferred electricity storage comprises one or more capacitor(s), coil(s), or battery/batteries or a combination of the aforementioned. In a preferred embodiment of the invention the storage capacity of the energy storage is configured so that it can hold, in an available form, at least 20%, particularly preferably at least 40%, particularly preferably at least 50%, particularly preferably at least 60%, particularly preferably at least 80%, particularly preferably at least 100%, particularly preferably at least 150%, particularly preferably at least 200%, particularly preferably at least 300% of the electrical energy required for the denaturation step of a passage of the amplification cycle of the PCR. With this embodiment of the invention, the situation of a power source having to be made available that can provide the electrical power required for the denaturation step over the whole duration of the denaturation step can be avoided. Instead, advantageously in the time between the denaturation steps, which is generally considerably longer than the duration of the denaturation step, the energy storage can be charged up. In this way it can be achieved that the device according to the invention can be equipped with a power source that is weaker in relation to its electrical output, for example with a weaker network device. If the electricity storage provides less than 100% of the energy required for the denaturation step of a passage of the amplification cycle of the PCR, it can advantageously be kept very small, but the remaining energy must then be provided by an additional, correspondingly dimensioned power source, for example a power network connection, during the denaturation step. Values of 100% and more are advantageous, as the power source supplying the capacitor(s) can then be kept correspondingly small and in addition the voltage does not collapse due to the load of the heating. In particular it can advantageously be achieved that a power source of the device according to the invention merely needs to have such dimensions that it is able to provide the amount of energy required for the denaturation step over the duration of an amplification cycle of the PCR, but not already during the significantly shorter denaturation time.

In a preferred embodiment of the invention, in at least one, preferably in at least three, particularly preferably in at least 10, particularly preferably in at least 20 of the passages of the amplification cycle of the PCR, the ratio between the electrical power consumption of the device and the capacity of the reaction vessel does not at any time during the PCR exceed 1 W/ml, preferably 0.5 W/ml, particularly preferably 0.25 W/ml, particularly preferably 0.1 W/ml. This limitation does not apply to possibly higher power consumption during the switch-on process of the device, as may be caused for technical reasons by switch-on currents. Such increased power consumptions are not regarded as power consumptions during the PCR and are not therefore taken into consideration here.

In a preferred embodiment of the invention the electricity storage is configured so that it can hold, in an available form, the energy for the denaturation steps of at least 5, particularly preferably at least 10, particularly preferably at least 20, particularly preferably at least 40, particularly preferably at least 100 passages of the amplification cycle of a PCT. In this way a device, in particular a portable device, can advantageously be created that can carry out one or even several polymerase chain reactions independently of another power source.

Preferred capacitors are high-capacity capacitators, preferably electrolyte capacitors or super-caps, particularly preferably having a low ESR value. Such capacitors can be obtained cost-effectively on the market and are easy to dimension. For example, using the equation $Q = \frac{1}{2}CU^2 \; \text{bzw.} \; C = \frac{2 \cdot Q}{U^2}$, it can be calculated that a capacitor with a capacitance of 2222 μF (microfarad) would be sufficient for the provision of 1 J (Joule) of electrical energy - which would suffice, with virtually loss-free conversion of the electrical energy into heat, according to an embodiment for the denaturation step of a reaction volume of 1 ml - with a voltage U of 30 V. However, the capacitor would then be completely discharged at the end of the heat pulse, so that in practice the use of a capacitor with at least 1.5 times higher capacitance is recommended if the power source cannot supply a considerable part of the power.

In a preferred energy storage according to the invention the capacitance of the capacitor, or, in the case of a plurality of capacitors, the sum of the capacitances of this plurality of capacitors, is greater than 100 μF, particularly preferably greater than 200 μF, particularly preferably greater than 500 μF, particularly preferably greater than 1 mF (millifarad), particularly preferably greater than 1.5 mF. In a preferred energy storage according to the invention the ratio between the capacitance of the capacitor, or, in the case of a plurality of capacitors, the sum of the capacitances of this plurality of capacitors, and the size of the reaction volume is greater than 0.01 mF/mL (millifarad per milliliter), particularly preferably greater than 0.1 mF/mL, particularly preferably greater than 1 mF/mL, particularly preferably greater than 5 mF/mL, particularly preferably greater than 10 mF/mL. With this embodiment of the invention, sufficient energy can advantageously be intermediately stored to achieve sufficient heating of the heating element in the denaturation step.

Particularly preferred batteries are high-current batteries or accumulators, in particular lithium-polymer accumulators, lithium-ions or lithium-iron phosphate accumulators. In one embodiment of the invention the battery/ batteries is/ are used with one or more capacitors. In another embodiment the battery/ batteries, is/ are particularly preferably lithium-iron phosphate accumulators, which are characterized by an advantageously low internal resistance, without additional use of a capacitor. It can be favorable according to the invention if the inlet lines running between capacitor(s) and/ or batteries and as far as the heating element are as short as possible, in order to reduce interference-causing inductances and ohmic resistances of the inlet lines.

Reaction vessel

A preferred device for the amplification of nucleic acids has a reaction vessel for receiving the reaction volume. Suitable reaction vessels of the method according to the invention can be conventional PCR reaction vessels such as PCR tubes or composites of PCR tubes (such as, e.g., so-called 8 stripes) or multiwell plates, but also, for example flat plates or other shapes/ forms that can be filled. The heating means can be brought into the reaction vessels, for example wires that pass through the walls of multiwell plates or PCR tubes, already during the production process (for example injection molding), or be added after the production process (as for example in the case of wires in the form of coils, which can be suspended in the individual wells of a multiwell plate).

Control device

A preferred device according to the invention comprises a control device, which applies electrical current to the heating means in order to heat the reaction volume. The control device is preferably configured so that, in at least one, preferably in at least three, particularly preferably in at least 10, particularly preferably in at least 20 of the passages of the amplification cycle of the PCR, the ratio between the electrical energy used in the denaturation step to heat the reaction volume and the capacity of the reaction vessel is less than 40 J/ml (millijoule per milliliter), particularly preferably less than 20 J/ml, particularly preferably less than 10 J/ml, particularly preferably less than 3 J/ml.

In a preferred device according to the invention the control device is configured so that at least one heating element of the heating means has in at least one direction an expansion of more than 1.5 $\mu$m. Particularly preferably each heating element of the heating means has, in at least one, particularly preferably in two, direction(s), an expansion of more than 1.5 $\mu$m. The expansion can for example be a length or a diameter of an elongate heating element. A preferred elongated heating element has a diameter of at least 1 $\mu$m, particularly preferably at least 2 $\mu$m, particularly preferably at least 5 $\mu$m. A preferred elongate heating element has a length of at least 0.1 mm, particularly preferably at least 1 mm, particularly preferably at least 2 mm. In the case of a network-form or honeycomb-formed heating element the expansion can also be, for example, also the thickness (meaning the expansion of the webs perpendicularly to the surface of the network-form or the honeycomb) or the diameter of the webs. Preferred webs have a thickness or a diameter of at least 1 $\mu$m, particularly preferably at least 5 $\mu$m, particularly preferably at least 10 $\mu$m.

The preferred control device is designed so that it allows, or increases, a current flow through the heating means at the start of the denaturation step - or at the start of each time interval of the denaturation step if the denaturation step is composed of a plurality of time intervals separated from each other, and again suppresses, or reduces, said current flow after the end of the denaturation step - or after the end of each time interval of the denaturation step - in order to bring about a current pulse. In the heating element the current pulse can be converted into a heat pulse. The preferred control device comprises a power source, which for its part can comprise for example a power network component, one or more battery (batteries) or accumulators or fuel cells. The preferred control device comprises one or more capacitors. The preferred control device comprises a switch, which, preferably with a selectable time duration, can switch on and off the current flow from the power source through the heating means. Suitable switches include MOSFETs, SSRs, very rapid relays and transistors. The control device can have one or more pulse or frequency generators, DACs or microcontrollers for time control.

Observation of the PCR and detection of PCR products

Preferably in the method according to the invention amplicons are detected or the original presence of the target nucleic acid in the sample is detected. This can be realized for example through gel electrophoresis after the PCR, hybridization of the amplicon to immobilized oligonucleotides, which are complementary to the amplicon or parts thereof, through a detection by means of, for example, fluorescent dyes or through a detection via electronic methods. In a further preferred embodiment, a real- time detection takes place already during the PCR in the reaction volume in order to observe the progress of the polymerase chain reaction with the aid of an optical method. For this, optical methods are particularly preferred, in particular fluorescent methods, for example in the TaqMan format. The relevant disclosure of US 5,210,015 and the publication by Holland et al., Proc Natl Acad Sci US A, 88(16), 1991, pages 7276 to 7280, are to be regarded as part of the present disclosure by virtue of reference thereto. In this method a specific fluorescent signal is produced during the PCR, which allows the real-time observation of the amplification reaction and even a quantification of the number of target nucleic acids originally used. Other real-time detection methods are also possible, for example the use of intercalating dyes such as SybrGreen and the use of molecular beacon probes. However, the invention also includes embodiments, in which the amplification of the nucleic acids is purely preparative in the sense that the amplicon is used further for example in a subsequent process.

A preferred device according to the invention comprises a light source, for example a semiconductor light source, for example a light emitting diode or a laser diode. Dyes can advantageously be excited in the reaction volume with the light source, preferably in order to observe the progress of the polymer chain reaction with the aid of an optical method.

A preferred device according to the invention comprises a light sensor, for example a semiconductor light sensor, for example a photodiode. Dyes can advantageously be detected in the reaction volume with the light sensor, preferably in order to observe the progress of the polymer chain reaction with the aid of an optical method. The light sensor can be equipped with one or more filters.

Where reference is made to "light" in connection with detection or observation methods relating to this invention, this includes all possible types and wavelengths of light that are suited for optical detection methods, in particular also those suited for excitation or detection of a fluorescent dye. The light is preferably visible light, but it can also be ultraviolet or infrared light. The light can be laser light.

By way of example, the first passage of the amplification cycle of the PCR can be carried out as follows in a method according to the invention: After the addition of nucleic acid molecules (hereinafter described as "target nucleic acids") to the reaction volume (and possibly the denaturation thereof by global heating) and the hybridization thereof to forward primers bonded. To one or more heating element, a polymerase elongates the forward primers and thereby produces complementary strands for the target nucleic acid. The denaturation, i.e. the separation of the molecules of the target nucleic acid from the elongated forward primers, is not realized by global heating of the whole reaction volume, but instead through a heat pulse, which is brought about by a current pulse through the heating element(s) of the heating means.

The subsequent second passage of the amplification cycle of the PCR can be realized in a similar way. The molecules of the original target nucleic acid hybridize again to forward primers bonded to one or more heating element(s) and the polymerase elongates the forward primers and hereby produces complementary strands for the target nucleic acid (or at least for a proportion of the target nucleic acid), In parallel, reverse primers (which are either freely suspended or also heating element-bonded) can bind to the elongated parts of the elongated heating element- bonded forward primers produced in the first passage of the amplification cycle of the PCR (the forward primers now constitute complementary strands to at least a proportion of the target nucleic acid) and the reverse primers are subsequently correspondingly elongated by the polymerase. In this way, for the first time genuine copies of at least a part of the original target nucleic acid are produced. The denaturation, i.e. the separation of the double strands produced through elongation by the polymerase (the double strands are in any case again bonded to the heating means) is realized once again through a heat pulse caused by the current pulse through the heating means. With effect from the third passage of the amplification cycle of the PCR, both the original target nucleic acid and also the nucleic acid strands produced by elongation of the primer sequences through the polymerase (depending on the embodiment: freely suspended in the reaction volume or heating element-bonded) as a template for the further amplification. They are amplified by hybridization to corresponding primers (according to the embodiment: freely in solution of bonded to a heating element), there is subsequent elongation by through the polymerase and then denaturation by means of a local heat pulse, which is brought about by a current impulse through the heating means. The last described passage of the amplification cycle of the PCR is repeated several times in order to produce further copies at least of parts of the target nucleic acid in each further passage of the cycle. The passages are repeated until as often as necessary until a sufficiently high number of copies at least of parts of the target nucleic acid are present in order to be able to carry out a detection of the amplification carried out or the original presence of the target nucleic acid in the sample. Using one of the methods described above, for example fluorescence method, the thus generated amplicons can be detected.

In a further exemplary embodiment of the invention a plurality of different target nucleic acids are amplified in parallel (also described as "multiplex-PCR"). For this, a plurality of primer pairs that are different from each other (in each case: forward and reverse primers) are necessary for each amplicon (wherein a primer can also serve as a primer for two amplicons, for example of different lengths, therefore being part of two primer pairs). A heating element can carry a plurality of primer pairs or in each case (at least) one primer made up of a plurality of primer pairs. However, a plurality of primers or primer pairs can also be distributed in such a way that different sub- portions of the heating element each carry only one primer pair or each carry only one partner of a primer pair. In an exemplary embodiment one (possible only one per primer pair or even each) primer sort or primer sequence can be present in both a heating element-bonded form and also freely suspended form in the reaction volume. A detection can be realized for example by using different dyes in such a way that different color signals are produced (with different wavelengths) can be assigned to the formation of different amplicons. Alternatively, however, different amplicons can also produce the same color signals, which cannot be differentiated. Different amplicons can also be differentiated, for example using gel electrophoresis or other methods.

Setting, or establishing, a global reaction temperature

The (global) elongation and hybridization temperature is kept constant in a preferred exemplary embodiment during the whole course of the PCR, for example by means of a conventional external heater, for example a temperature-regulating block, or through a constant (or regulated) excitation of the heating element by means of a constant (or regulated) offset current through the heating element.

The regulation of the (global) elongation and hybridisation temperature or the heating temperature can be realised on a temperature sensor in the reaction volume, in one of a plurality of reaction volumes, individually for each individual reaction volume through a respective sensor in the respective reaction volume, by a sensor outside of the reaction volumes or through a sensor in the heater or a recording device for the reaction volume. In one embodiment the (global) elongation and hybridization temperature for all reaction volumes is the same, in a further embodiment the (global) elongation and hybridization temperature can differ for the different reaction volumes. In a further embodiment the (global) elongation and hybridization temperature can be varied or changed during the duration of the PCR or before or after the PCR. In one embodiment the heater can also consist of a plurality of parts, for example from a bottom part and a top part, wherein the top part has a somewhat higher temperature than the bottom part in order to avoid condensation on the walls of the reaction volumes. The temperature difference between the top and bottom part of the heater is preferably between 1°C and 30°C, particularly preferably between 2°C and 20° C and most particularly preferably between 3°C and 15°C.

In one embodiment the global heating of the reaction liquid in the reaction vessel through electrical heating of the heating means can account for a part of the heat input or all of the heat that is required to reach the desired elongation and hybridization temperature. This can for example be achieved by the duty cycle and/ or the continuous electrical current (or voltage at) the heating element(s) of the heating means being selected so that the global heating of the reaction liquid in the reaction vessel in thermal equilibrium or at the end of the PCR or at the start of the PCR or during a large part of the duration of the PCR leads to the desired elongation and hybridization temperature in the reaction liquid. The external heating (i.e. the element for the heat input that does not come through the heating element) can thereby have smaller dimensions or be completely omitted.

Preferred temperatures according to the invention for the combined hybridization and elongation temperature are preferably between 30°C and 85°C, particularly preferably between 40°C and 80°C, particularly preferably between 50°C and 75°C and most particularly preferably between 55°C and 72°C.

In one embodiment, a global heating step (with a global temperature greater than the later hybridization and elongation temperature) can take place before the first actual passage of the PCR cycle, wherein said global heating step can serve for initial denaturation of the double-stranded present target nucleic acid (DNA or RNA or other nucleic acid) and / or for thermal activation of other reaction partners of the PCR such as for example (hot-start) polymerases and/ or for deactivation of constituent parts of the reaction volume, which are to be active before the PCR but not during the PCR (such as for example the enzyme Uracil-DNA-glycosylase).

In one embodiment, a further global heating step with a lower global temperature than in the abovementioned global heating step can take place before this global heating step, wherein the further global heating step can be utilized for example, an enzyme being given off, a reaction taking place before the PCR (such as for example the overwriting of RNA into DNA by a transcriptase enzyme).

Spatial heat spreading during and after the denaturation step

As soon as the current flow through the heating element in the denaturation step has begun, the heating element begins to heat up. As most current-conducting materials (in particular metals) also convey heat very well, the heating element heats approximately homogeneously over the duration of the heat pulse. At the surface of the heating element, which is surrounded in a preferred exemplary embodiment by the aqueous reaction volume, the heat is transferred to the reaction volume, where it spreads. The spreading of a thermal field is realized in the reaction volume through heat diffusion, for which a root-form rule applies.

$$d \approx \sqrt{D \cdot t}$$

Equation 1

wherein d describes the path distance covered by a heat front after a time t along a spatial direction in a reaction volume with temperature conductivity D and is to be referred to below as "heat diffusion range". This means, that for a heating duration of for example 100 $\mu$s, the heat generated in the heating element can diffuse far into the reaction volume with a typical temperature diffusivity (also known as "temperature conductivity") of D $\approx$ 1.6 * 10$^{-7}$ m$^2$/s in terms of value

$$d \approx \sqrt{\frac{1.6 \cdot 10^{-7} m^2}{s} \cdot 10^{-4} s} \approx 4 \mu m$$

In other words, the heat generated in the heating element for example by ohmic losses has spread after 100 $\mu$s into the reaction volume surrounding the heating element, namely with a magnitude in the range of 4 $\mu$m.

Through the spatial spread of the heat corresponding to the above equation the amount of heat brought is distributes over an increasingly large volume so that, perpendicular to the surface of the heating element, which is hotter by a temperature $\Delta$T than the global average temperature, a temperature gradient of the value $\Delta$T/d results, which facilitates the heat transport. A more precise estimation of the spatial heat expansion during and after the heat pulse for the respective geometry of the heating element can be achieved by finite element methods such as, for example, with commercial solutions like for example Comsol, which facilitate a numerical solution of the heat diffusion equation. The reaction volume within a layer with the thickness of a heat diffusion range is preferably heated around the heating means by at least more than 10°C.

In a preferred embodiment of the invention the heat diffusion range in at least one, preferably in at least three, particularly preferably in at least 10, particularly preferably in at least 20 of the passages of the amplification cycle of the PCR, at the end of the denaturation step is preferably between 0.05 $\mu$m and 200 $\mu$m, particularly preferably between 0.2 $\mu$m and 100 $\mu$m, particularly preferably between 0.5 $\mu$m and 50 $\mu$m and most particularly preferably between 1 $\mu$m and 25 $\mu$m. It is an achievable advantage of this embodiment of the invention that on the one hand a sufficient spatial expansion of the heated area perpendicular to the surface of the heating element can be achieved and that the PCR amplicons formed on the heating element, which typically have a length of between 0.02 and 3 $\mu$m (correspondingly roughly between 60 and 10000 base pairs), can be heated as homogeneously as possible and thus denatured, and that the heat diffusion range is not so large that the volume ratio of the heating-up zone to the unheated passive volume becomes too low. In the sense of the present invention the "heating-up zone" is the part of the reaction volume, in which the heat can diffuse during the denaturation step. The expansion of the expansion of the heating-up zone perpendicular to the surface of the heating element can be estimated approximately through the heat diffusion range defined above. The volume of the reaction liquid that is not in the heating-up zone is referred to as "unheated passive volume". This means for example in the case of a cylindrical heating element (for example a heating wire) that the heating-up zone can be estimated as the volume located at the distance of a heat diffusion range d from the cylinder surface (i.e. the cylinder shell with thickness d). If the heating element is for example an elongate cylinder with radius r and length 1 (for example a wire), the volume of the heating-up zone can be roughly estimated as

$$V_{AHZ} \approx \pi \cdot l \cdot ((r + d)^2 - r^2)$$

Equation 2

In a preferred embodiment of the invention the volume ratio of the heating-up zone to the unheated passive volume in at least one, preferably in at least three, particularly preferably in at least 10, particularly preferably in at least 20 of the passages of the amplification cycle, at the end of the denaturation step is less than 10%, preferably less than 5%, particularly preferably less than 2%, particularly preferably less than 1%, particularly preferably than 0.5%, particularly preferably less than 0.25% and most particularly preferably less than 0.1%. With this embodiment of the invention a high localization of the heat can be achieved, which means that the amount of heat brought in the denaturation step can spread after the denaturation step to the unheated passive volume. As the unheated passive volume is many times greater than the heating-up zone, the distribution of the amount of heat over the whole reaction volume (= heating-up zone+ unheated passive volume) can lead to a preferably negligible global temperature increase of the whole reaction volume, so that a very rapid cooling of the heating-up zone is possible and in addition the cooling process is (extensively) independent from a discharge of the heat to outside of the sample

Estimation of the local temperature increase in the denaturation step

For typical denaturation temperatures of the double-stranded nucleic acid of between 85°C and 98°C, in one embodiment a local temperature lift with respect to the combined hybridization and elongation temperature of roughly between 20°C and 40°C must be reached on the surface of the heating elements and over the length of the double-stranded nucleic acid to be denatured.

In a preferred embodiment of the invention the temperature of the area of the heating means that is in contact with the reaction volume is, in at least one, preferably in at least three, particularly preferably in at least 10, particularly preferably in at least 20 of the passages of the amplification cycle of the PCR, during the denaturation step, is between 70°C and 250°C, particularly preferably between 75°C and 150°C, particularly preferably between 80°C and 120°C, most particularly preferably between 80°C and 100°C.

In a preferred embodiment of the invention the average temperature of the area of the heating means that is in contact with the reaction volume is over 100°C. With this embodiment of the invention, a particularly rapid separation of the double strand is advantageously possible. This embodiment of the invention utilizes the fact that a short-term overheating of the reaction volume is also possible on the surface of the heating means without vapor bubbles forming (inter alia, on account of the high Laplace pressure due to the curvature of the surface of the heating element - see specialist literature for Young-Laplace equation.

In the case of complex geometries of the heating means and/ or in order to ensure a high precision, the use of finite element methods (for example Comsol) is advisable, in order to determine the temperature of the heating element as a function of the electrical operating parameters. In such simulations, in the simplest case a constant volumetric heating density can be assumed or a current flow through the heating means can be simulated. In many cases, however, the temperature increase brought about by the heating means can be estimated by a simple calculation, which is set out by way of example below.

Firstly, the amount of heat that is released in a conductor with a current flowing through it is determined. The electrical power P, which is available during the electrical heat pulse for heating the heating element, is calculated from the resistance of the heating element R and the voltage U supplied at the heating element as P = U2/R. The amount of heat Q released in the heating element is then the electrical power P times the heating duration theat,

$$Q = U^2 \cdot t_{heat} / R$$

Equation 3

wherein, here, a temporally constant voltage and constant resistance were assumed over the time duration of the heat pulse. If this is not the case, then the following applies:

$$Q = \int_0^{t_{heat}} U(t)^2 / R(t) \cdot dt$$

If the heating means is made up in one embodiment (in portions) of homogeneous conductors with constant cross-section area, then the resistance of a homogeneous conductor R can be calculated from its cross-section area A and its length I as well as the specific conductivity a of this conductor element as

$$R = \frac{l}{A \cdot \sigma}$$

Equation 4

Typical values for the specific conductivity are shown in tables in the specialist literature and are as follows for typical materials such as gold: $\sigma_{Au} = 4.6 \cdot 10^7 \frac{A}{V \cdot m}$, for tungsten: $\sigma_W = 1.9 \cdot 10^7 \frac{A}{V \cdot m}$ and for V2A (stainless steel):

$$\sigma_{V2A} = 1.4 \cdot 10^6 \frac{A}{V \cdot m}.$$

If it is assumed that the duration of the heat pulse is so short that the energy in initially only heats the heating element(s) and the heating-up zone, the local temperature increase of the heating means, which is indeed to be heated to the denaturation temperature, can be estimated as follows:

$$\Delta T_{Local} \approx \frac{Q}{c_{MH} \cdot m_{MH} + c_{AHZ} \cdot m_{AHZ}}$$

Equation 5

Here, $C_{MH}$ describes the specific heat capacity (per unit of mass xx) of the heating element and $m_{MH}$ describes the mass thereof and also $c_{AHZ}$ the (mass-related) specific heat capacity of the heating-up zone (which is $c_{AHZ}$ = 4.2 J/(°C*g) for the aqueous PCR solution) and $m_{AHZ}$ is the mass of the heating-up zone. The above approximation is all the more precise, the smaller the heat capacity of the heating-up zone in comparison with the heat capacity of the heating element. This is due to the fact that the above equation does not take into consideration that the temperature rapidly falls in the heating-up zone (i.e. a solid temperature gradient forms around the heating element).

If the heating duration is selected to be so short that the size of the heating-up zone remains very small (significantly smaller than the heating element itself) and therefore its heat capacity is negligible with respect to the heat capacity of the heating element, the above equation can be simplified to:

$$\Delta T_{Local} \approx \frac{Q}{c_{MH} \cdot m_{MH}}$$

Equation 6

While Equation 6 can only be applied in special cases and for very short heating durations, Equation 5 can be used as an approximation for determining the local temperature on the surface of the heating element(s), wherein it must be checked in each case from the geometry and the actual current flow how, above all, the mass of the heating-up zone can be calculated. In many cases, the mass of the heating-up zone can be estimated from its volume by taking into consideration the geometry of the heating element and also the heat diffusion range (see above).

The case that is particularly relevant according to the invention will be considered below, wherein the heating means is formed at least in portions (approximately) cylindrically and at least in portions is homogeneous and has a constant cross- section. The following calculation is to be regarded as an example for a cylindrical geometry of the heating means and can very easily also be transferred to other geometries. The mass of the heating element in Equation 5 can then be calculated from its volume and density:

$$m_{MH} = P_{MH} * \cdot A \cdot * l,$$

wherein A is the cross-sectional surface area and 1 is the length of the heating element (or the portion thereof considered.). The amount of heat Q can then be calculated with Equation 3 and Equation 4.

With Equation 2, which describes approximately the volume of the heating-up zone, which surrounds an (approximately) cylindrical conductor portion with radius r and length 1, the mass of the heating-up zone can be estimated in Equation 5 as :

$$m_{AZ} = V_{AHZ} \cdot p_{AHZ} = \pi \cdot l \cdot ((r + d)^2 - r^2) \cdot p_{AHZ},$$

wherein the density of the heating-up zone in the reaction volume is equal to the density of water ($P_{AHZ} \approx P_{H2O} \approx 1g * cm^{-3}$). Therefore, for a heating element that is (approximately) cylindrically formed and at least in portions is homogeneous and has a constant cross-section, the following estimation can be given using Equation 2, Equation 3, Equation 4, Equation 5:

$$\Delta T_{Local} \approx \frac{\frac{U^2 \cdot t_{heat}}{l}}{\frac{A \cdot \sigma}{c_{MH} \cdot p_{MH} \cdot A \cdot l + c_{AHZ} \cdot \pi \cdot l \cdot ((r + d)^2 - r^2) \cdot p_{AHZ}}}$$

For a cylindrical conductor portion, the cross-sectional area can be calculated form the radius A=$\pi r^2$, so that, together with Equation 1, the following simplification is given:

$$\Delta T_{Local} \approx \frac{U^2 \cdot \sigma}{l^2} \cdot t_{heat} \cdot \frac{1}{c_{MH} \cdot p_{MH} + c_{AHZ} \cdot p_{AHZ} \cdot \left( \left( r + \sqrt{D \cdot t_{heat}} \right)^2 - r^2 \right) / r^2}$$

Equation 7

With the above equation, the temperature to which an (approximately) cylindrical conductor is heated during the heating duration $t_{heat}$ can thus be approximately estimated, in order to preferably be able to determine the parameters for achieving the denaturation temperature. If only a portion of the heating element is taken into consideration (for example because the heating element consists of a complex geometrical series arrangement of conductors), it is obvious that, for the voltage, only the voltage that drops over the respectively considered conductor is relevant.

The first factor $\frac{U^2 \cdot \sigma}{l^2}$ in the above equation is thereby the volumetric heating density, which is described as q below, i.e. $q = \frac{U^2 \cdot \sigma}{l^2}$. This in turn means that the temperature increase in Equation 8 is proportional to the volumetric heating density.

If the heating element(s) is/are for example designed as wires from gold (Au) with a length of the wire l = 0.1 m and also a radius of the wire of r = 12.5 $\mu$m and the material parameters

$$p_{MH} = p_{Au} = 19200 \frac{kg}{m^3}, c_{MH} = c_{Au} = 125 \frac{J}{kg \cdot °C} \text{ and } \sigma = \sigma_{Au} = 4.6 \cdot 10^7 \frac{A}{Vm}$$

based on a m voltage of U = 10.5 V spacing before V and a heating duration of $t_{heat}$ =200 $\mu$s according to Equation 7 this results in a local temperature increase on the heating element of

$$\Delta T_{local} \approx 14.4°C. \text{ (when using } p_{AHZ} = p_{H_2O} = 1000 \frac{kg}{m^3}, c_{AHZ} = c_{H_2O} = 4200 \frac{J}{kg \cdot °C},$$

which are typical values for the reaction volume contained in the heating-up zone). From the operating parameters used, the volumetric power and heating density q can be calculated with $q = \frac{U^2 \cdot \sigma}{l^2} \approx 5 \cdot 10^{11} W/m^3$ , so that a comparison with the results of the 1 finite element simulations in Fig. 2B of WO 2018/073435 is possible. This shows, after 200 $\mu$s heating durations for a cylindrical heating wire at the said heating density of 5 * $10^{11}$ W/m$^3$, a heating of just about 17°C.

Provision of the electrical power and energy density

In a preferred embodiment of the invention, in at least one, preferably in at least three, particularly preferably in at least 10, particularly preferably in at least 20 of the passages of the amplification cycle of the PCR, the average volumetric power density of the heating means is greater than $10^9$ W/m$^3$ ,preferably greater than $10^{10}$W/m$^3$, particularly preferably greater than $10^{11}$W/m$^3$. It is an achievable advantage of this embodiment of the invention that the heating element is heated sufficiently quickly even with short duration of the heat pulse.

In a preferred embodiment of the invention, in at least one, preferably in at least three, particularly preferably in at least 10, particularly preferably in at least 20 of the passages of the amplification cycle of the PCR, the average specific power density of the heating means is preferably less than $10^{16}$ W/m$^3$, particularly preferably less than $10^{15}$ W/m$^3$ and particularly preferably less than $10^{14}$ W/m$^3$. With this embodiment of the invention, damage to the heating elements can advantageously be avoided.

In the case of a heating resistor being used as a heating element, the specific power density q, which is generated in the heating element, is given by

$$q = \frac{U^2 \cdot \sigma}{l^2}$$

i.e. a voltage U that is as high as possible, a conductivity $\sigma$ that is as high as possible and a short length 1, over which the voltage drops in the heating element lead to a high specific power density. The entire electrical power requirement resulting for the provision of the heat pulses is thus calculated from the required volumetric power density and the combined volume of all heating elements of the heating means, wherein it depends, inter alia, upon the reaction volume that is to be processed.

Global heating of the sample through the local heating step for the denaturation

In a preferred embodiment of the invention, current pulses through the heating means are selected so that only the heating means and the reaction volume in the direct vicinity of the surface of the heating means are significantly heated, thus a merely local heating takes place. The amount of heat Q brought in the course of the whole denaturation step is produced locally in the heating means and is distributed initially over the heating means itself and the direct vicinity thereof, as the discharge of the heat through diffusion takes place only gradually, as explained below. This means that the amount of heat Q brought is an amount of energy that is initially distributed over a very small volume and, in time, spreads ("flows") into the surrounding reaction volume. Provided that the amount of heat (often also only described just as the "heat") is still spatially concentrated in the heating element and its direct vicinity, it brings about a substantial temperature increase there. As soon as this amount of heat is distributed over an increasingly large volume, however, it also brings about a temperature increase there, but which is correspondingly smaller, as the originally brought amount of heat naturally remains constant (if only the liquid volume of the reaction volume is considered, the temperature decreases inversely proportionally to the volume, over which the amount of heat is distributed).

It is only after a certain time, hereinafter referred to as the "sample thermalisation time" and defined in the following paragraph, that the amount of heat is distributed to the whole reaction volume and causes a global temperature increase there.

Depending on how well insulated the reaction volume is, or how well it is coupled to an external thermic tank, the brought amount of heat can remain in the reaction volume (with very good insulation, which then leads to a gradual, slight increase in the global temperature of the reaction volume with each passage of the amplification cycle of the PCR) or, in the case of good thermic thermal contacting of the reaction volume, the heat flows away, so that the reaction volume goes back to the original temperature (before the heating step). In practice, it is mostly the case that a part of the brought amount of heat flows away in the time between two denaturation steps so that the global temperature of the reaction volume increases slightly (typically less than 3°C) over the first passages of the amplification cycle of the PCR until an equilibrium state has formed and, for each cycle, the same amount of heat is brought in as the amount of heat that flows away.

The sample thermalization time is the time until the brought amount of heat has spread from the heating means to the whole reaction volume. The sample thermalization time can be estimated by initially determining the point(s) at the greatest distance $d_{max}$ from the nearest heating element (typically, in many cases, these points lie on the surfaces that delimit the reaction volume). The sample thermalization time is then the time taken until the heat diffusion range is equal to $d_{max}$, i.e. in terms of image, until the heat that is generated in the heating elements has diffused into the last "corner" of the reaction volume. If, for example, the reaction volume is cylindrical with a radius of 1.01 mm and the heating element consists of a single cylindrical wire with a radius of 0.01 mm, which runs concentrically through the axis of the cylinder, the maximum distance that a point in the reaction volume can be from the nearest (in this case the only) heating element is $d_{max}$ = 1 mm. With Equation 1, a heat diffusion range of 1 mm is produced after 6.3 s, so that in this special case the sample thermalization time is approximately 6.3 s.

The MGTE, which is the maximum increase in the average temperature taking place through the denaturation step, can be estimated as follows from amount of heat Q and the heat capacitance that is brought through the heating step into reaction volume, with $MGTE \leq \frac{Q}{C}$. With the density p of the reaction volume, its volume V, its specific heat capacity c and with the aid of the correlation C = c * p * V it can be estimated that $MGTE \leq \frac{Q}{c \cdot p \cdot V}$. The less-than sign is substantiated in that the heat capacity of the heating means and the reaction container is not taken into consideration here. The density and heat capacity of the reaction volume is generally substantially that of water, i.e. p = lg * cm$^3$ and c = 4.2 J * °C$^{-1}$ * g$^{-1}$. The amount of heat Q can be determined from the electrical operating parameters: If the heat pulse $t_{heat}$ continues and if the voltage U and the current I during the heat pulse are constant, then Q = U * I * $t_{heat}$ (Insofar as current and voltage are changeable over time, the following applies:

$$Q = \int_0^{t_{heat}} U(t) \cdot I(t) \cdot dt)$$

This means in the first case that the upper limit for the MGTE can be determined by the equation

$$MGTE \leq \frac{U \cdot I \cdot t_{heat}}{4.2\,J \cdot cm^{-3} \cdot V}$$

Equation 8

(the volume V is then to be indicated in milliliters). Here, of course, only the voltage U that drops via the heating means in the reaction volume V is to be considered, and also the current I that actually flows through the heating means in the reaction volume V. (This means: the voltage drop in inlet lines would for example not have to be considered.)

The MGTE can be experimentally determined by the temperature of the reaction volume being taken before and after a single denaturation step, wherein in the latter case it is only after the sample thermalization time that the physical measurement of the temperature in the reaction volume is carried out. The difference between the two measured temperatures is then equal to the MGTE. This procedure is advantageous according to the invention, as a complete thermalization of the reaction volume, i.e. an even distribution of the heat in the reaction volume is ensured and the temperature sensor does not detect the temperature of the heating-up zone, for example randomly). This measurement of the MGTE can for example in practice be detected with a temperature sensor, preferably with a particularly small heat capacity of its own, which is brought into the reaction volumes.

The following clauses are also comprised by the spirit and scope of the underlying invention:

1. Diagnostic method for the detection of at least one virus, preferably at least one DNA virus and/or RNA virus, in at least one ex-vivo sample of one or more animals, comprising the step

(a) isolating at least one sample of such one or more animals,
(b) amplifying any present viral genomic nucleic acid contained in such isolated at least one sample by means of a polymerase chain reaction in a reaction volume, wherein the reaction volume is heated through the use of electrical energy, characterised in that in at least one of the passages of the amplification cycle of the polymerase chain reaction, the ratio of the electrical energy used in the denaturation step for heating the reaction volume to the size of the reaction volume is less than 20 Joule per millilitre (mL); and

(c) detecting the presence of the amplified viral genomic nucleic acid contained in such isolated at least one sample of such at least one animal.

2. The diagnostic method according to clause 1, wherein a heating means consisting of one or a plurality of electrically contacted heating elements, which are in contact with the reaction volume, heats the reaction volume, characterised in that in at least one of the passages of the amplification cycle of the polymerase chain reaction, the heating means supplies to the reaction volume less heat generated in the denaturation step than $C_R * 5°C$, wherein $C_R$ is the heat capacity of the reaction volume during the heating by means of the heating means.

3. The diagnostic method according to clause 2, characterised in that, in at least one of the passages of the amplification cycle of the polymerase chain reaction, the maximum increase of the average temperature of the reaction volume, taking place through the denaturation step, is less than 10°C.

4. The diagnostic method according to any one of clauses 2 to 3, characterised in that in at least one of the passages of the amplification cycle of the polymerase chain reaction, the heating means supplies to the reaction volume less heat generated in the denaturation step than $C_R * 5°C$, wherein $C_R$ is the heat capacity of the reaction volume during the heating by the heating means and, while the heating means is heating the sample, a temporally stable temperature is not established on at least 10% of the contact area of the heating means with the reaction volume.

5. The diagnostic method according to any one of clauses 2 to 4, characterised in that the ratio between the surface of the heating element(s), which is in contact with the reaction volume, and the reaction volume is greater than 0.1 per metre.

6. The diagnostic method according to any one of clauses 2 to 5, characterised in that the heat supply through the heating means varies during the polymerase chain reaction.

7. The diagnostic method according to any one of clauses 1 to 6, characterised in that in at least one of the passages of the amplification cycle of the polymerase chain reaction, the cycle duration $t_c$ is shorter than 60 seconds.

8. The diagnostic method according to any one of clauses 1 to 7, characterised in that the duration of the polymerase chain reaction $t_{PCR}$ is shorter than 45 minutes.

9. The diagnostic method according to any one of clauses 1 to 8, characterised in that the at least one virus is a DNA virus, such as African Swine Fever Virus (ASFV), Porcine Parvovirus and Porcine Circovirus.

10. The diagnostic method according to any one of clauses 1 to 8, characterised in that the at least one virus is an RNA virus, such as Swine Influenza Virus (SIV), Classical Swine Fever Virus and Sapovirus.

11. The diagnostic method according to any one of clauses 1 to 11, characterised in that the polymerase chain reaction according to step (b) is carried out with the nucleic acid sequences (forward and reverse primers) selected from the group consisting of: SEQ ID NOS: 1, 2, 7, 8, 11, 12, 15, 16

12. The diagnostic method according to clause 11, characterised in that the polymerase chain reaction according to step (b) is carried out with the nucleic acid sequences (forward and reverse primers) selected from the group consisting of: preferably SEQ ID NO: 1 + SEQ ID NO: 2, SEQ ID NO: 7 + SEQ ID NO: 8, SEQ ID NO: 11 + SEQ ID NO: 12 or SEQ ID NO: 15 + SEQ ID NO: 16.

13. The diagnostic method according to any one of clauses 11 to 12, characterised in that the polymerase chain reaction is a solid-phase polymerase chain reaction.

14. The diagnostic method according to clause 13, characterised in that one of the nucleic acid sequences (forward and reverse primer) selected from the group consisting of: SEQ ID NO: 1 + SEQ ID NO: 2, SEQ ID NO: 7 + SEQ ID NO: 8, SEQ ID NO: 11 + SEQ ID NO: 12 or SEQ ID NO: 15 + SEQ ID NO: 16, is immobilized on the heating elements according to clause 2.

15. The diagnostic method according to clause 11, characterised in that such nucleic acid sequences (forward and reverse primers) are directed to ASFV and selected from the group consisting of: SEQ ID NOS: 1, 2, 7, 8.

16. The diagnostic method according to clause 11, characterised in that the nucleic acid sequences (forward and reverse primers) are directed to SIV and selected from the group consisting of: SEQ ID NOS: 11, 12, 15, 16.

17. The diagnostic method according to any one of clauses 1 to 13, characterised in that the detection according to step (c) is carried out with one or more of the nucleic acid sequences (TaqMan probes) selected from the group consisting of: SEQ ID NOS: 3, 4, 5, 6, 9, 10, 13, 14, 17, 18.

18. The diagnostic method according to clause 17, characterised in that such nucleic acid sequences (TaqMan probes) are directed to ASFV and selected from the group consisting of: SEQ ID NOS: 3, 4, 5, 6, 9, 10.

19. The diagnostic method according to clause 17, characterised in that such nucleic acid sequences (TaqMan probes) are directed to SIV and selected from the group consisting of: SEQ ID NOS: 13, 14, 17, 18.

20. The diagnostic method according to any one of clauses 1 to 19, characterised in that the at least one ex-vivo sample is a blood sample or a tissue sample.

21. The diagnostic method according to any one of clauses 1 to 20, characterised in that the one or more animals is a mammal, preferably swine, more preferably a domestic pig.

22. Use of the amplification method according to any one of clauses 1 to 21 for the detection of at least one virus, preferably at least one DNA virus and/or RNA virus, in at least one ex-vivo sample of one or more animals.

**EXAMPLES**

The following examples serve to further illustrate the present invention; but the same should not be construed as a limitation of the scope of the invention disclosed herein.

EXAMPLE 1 - Detection of DNA virus (African Swine Fever Virus, ASFV)

PCA technology enables ultra-fast detection of nucleic acids, usually within 10 minutes, but as fast as two minutes. To facilitate fast overall reaction times, amplification-cycle durations of 2-3 seconds, short amplicons (< 120 bp) within conserved gene regions are selected for assay development. Since PCA requires no global heating steps, primer-dimers and secondary structures are avoided through selective primer design. Primer analysis is performed *in silico* using an oligo analyzer tool provided by Integrated DNA Technologies (IDT). For ASF virus detection, p72 is selected as the target gene. This gene encodes a conserved capsid protein of the dsDNA virus. The sequence of ASF1 reverse primer is identical to that published by King et al., J Virol Methods 2003, 107(1), 53-61. By intending to design an amplicon of 80-120 bp, allowing short cycle durations, a suitable region for the forward primer is selected. The ASF1 forward primer partially overlaps with the target region of the hydrolysis probe designed by King et al., J Virol Methods 2003, 107(1), 53-61. In parallel, an alternative completely novel assay ASF2 is also designed. For both ASF1 and ASF2, several TaqMan probes are designed.

The below depicted primer probe sets can be used analogously to DE 10 2016 120 124.3 and DE 10 2018 103 215.3 for the detection of ASFV by means of a PCR assay based on denaturation, which is mediated by locally heated heating elements in the reaction solution.

TaqMan probes are modified with fluorophores and quenchers. The combinations FAM-BHQ1 and Cy5-BHQ2 are utilized. To generate functionalization primers that attach covalently to the heating elements (microcyclers), a thiol-modification, a poly-A sequence and 1xSpacer9 sequence are added to the 5'-end of the respective primer sequences.

All sequences are illustrated in 5' - 3' orientation.

ASF-1

Forward Primer: GATACCACAAGATCAGCCGT (SEQ ID NO: 1)
Reverse Primer: GGAGGAATACCAACCCAGT (SEQ ID NO: 2)
TaqMan-Probe 1: CCACGTAATCCGTGTCCCAACTAA (SEQ ID NO: 3)

TaqMan-Probe 2: TGATAGACCCCACGTAATCCGTGTCC (SEQ ID NO: 4)
TaqMan-Probe 3: TAATCCGTGTCCCAACTAATATAAAATTCTCT (SEQ ID NO: 5)
TaqMan-Probe 4: TCATATTAACGTATCCAGAGCAAGAGAA (SEQ ID NO: 6)

As for ASF-1, the Forward-Primer (SEQ ID NO: 1) is based on King et al., J Virol Methods 2003, 107(1), 53-61.

Either Forward- or Reverse-Primer are immobilized on the heating elements via thiol modification. The respective primer is modified on its 5'-end as follows: 5'thiol - poly-A sequence - Spacer9 - primer-sequence. Hereby, the 5'-thiol moiety functions as covalent coupling agent for coupling the respective primer to the gold surface of the heating elements, the poly-A sequence functions as "spatial buffer" between the surface of the heating elements and primer sequence and can e.g. also improve the steric accessibility (being e.g. composed of 5 to 45 adenine bases), whereas Spacer9 is an abasic modification which can prevent the overwriting or complementation of the poly-A sequence through the polymerase.

TaqMan-Probe 1 (SEQ ID NO: 3), TaqMan-Probe 2 (SEQ ID NO: 4), TaqMan-Probe 3 (SEQ ID NO: 5) and TaqMan-Probe 4 (SEQ ID NO: 6) are possible variants for the real-time detection of the amplification reaction.

An exemplary setting for the ASF1 assay is shown below:

| Parameter | Settings |
| --- | --- |
| Initial Thermalizing [s] | 120 |
| Number of Cycles | 600 |
| Cycle Time [s] | 2 |
| Lid Temperature [°C] | 70,5 |
| Base Temperature [°C] | 63,5 |
| Heating [$\mu$s] | 300 |

Here, the Initial Thermalizing is the time interval that the reaction volume is in contact with the heating blocks, before the pulsing for denaturation starts. During that time interval the reaction volume thermalizes to the combined annealing/elongation temperature. Number of cycles is the number of pulses applied for denaturation; Cycle Time is the temporal spacing between two pulses. Lid Temperature is the temperature of the metal block that heats the lid of the reaction volumes to avoid condensation, Base Temperature is the temperature of the metal block that heats the reaction volume from underneath to set the combined annealing/elongation temperature of the reaction volume. Heating is the duration of each electrical heating pulse.

ASF-2

Forward Primer: CTCGGTGTTGATGAGGATTT (SEQ ID NO: 7)
Reverse Primer: ACCACCACGATGAAAAACTAA (SEQ ID NO: 8)
TaqMan-Probe 1: TCGGAGATGTTCCAGGTAGGTTT (SEQ ID NO: 9)
TaqMan-Probe 2: TCGGAGATGTTCCAGGTAGG (SEQ ID NO: 10)

Either Forward- or Reverse-Primer are immobilized on the heating elements via thiol modification. The respective primer is modified on its 5'-end as follows: 5'thiol - poly-A sequence - Spacer9 - primer-sequence. Hereby, the 5'-thiol moiety functions as covalent coupling agent for coupling the respective primer to the gold surface of the heating elements, the poly-A sequence functions as "spatial buffer" between the surface of the heating elements and primer sequence and can e.g. also improve the steric accessibility (being e.g. composed of 5 to 45 adenine bases), whereas Spacer9 is an abasic modification which can prevent the overwriting or complementation of the poly-A sequence through the polymerase.

TaqMan-Probe 1 (SEQ ID NO: 9) and TaqMan-Probe 2 (SEQ ID NO: 10) are possible variants for the real-time detection of the amplification reaction.

**EXAMPLE 2** - Detection of RNA virus (Swine Influenza Virus, SIV):

Swine Influenza Virus (SIV), a (-) ssRNA virus, is selected as RNA pathogen for later RNA-DNA-duplex PCA. The gene encoding Nucleoprotein (NP), a protein responsible for packaging of the viral genome, is chosen as target region. The primer design is chosen as needed in order to achieve an amplicon length of 80-120 bp.

The below depicted primer probe sets (SEQ ID NOS: 11 to 18) can be used analogously to DE 10 2016 120 124.3 and DE 10 2018 103 215.3 for the detection of SIV by means of a PCR assay based on denaturation which is mediated by locally heated heating elements in the reaction solution.

Since SIV is an RNA virus, the enzyme reverse transcriptase is added to the amplification solution in order to transcribe the original RNA into cDNA. Optionally, such reverse transcription can also take place in a preceding reaction step with its own reaction chemistry.

TaqMan probes are modified with the fluorophores and quenchers with either a FAM-BHQ1 combination or a Cy5-BHQ2 combination. For the functionalization primers, a thiol-modification, a poly-A sequence and 1xSpacer9 are added to the 5'-end of the respective primers.

All sequences are illustrated in 5' - 3' orientation.

SIV-1

    Forward Primer: GACGAAAAGGCAACGAACC (SEQ ID NO: 11)
    Reverse Primer: GCATTGTCTCCGAAGAAATAAGA (SEQ ID NO: 12)
    TaqMan-Probe 1: TACTCATGTCAAAGGAAGGCACG (SEQ ID NO: 13)
    TaqMan-Probe 2: ATGTCAAAGGAAGGCACGAT (SEQ ID NO: 14)

Either Forward- or Reverse-Primer are immobilized on the heating elements via thiol modification. The respective primer is modified on its 5'-end as follows: 5'thiol - poly-A sequence - Spacer9 - primer-sequence. Hereby, the 5'-thiol moiety functions as covalent coupling agent for coupling the respective primer to the gold surface of the heating elements, the poly-A sequence functions as "spatial buffer" between the surface of the heating elements and primer sequence and can e.g. also improve the steric accessability (being e.g. composed of 5 to 45 adenine bases), whereas Spacer9 is an abasic modification which can prevent the overwriting or complementation of the poly-A sequence through the polymerase.

In case the immobilized primer is also to be used for the extraction of RNA analogously to DE 10 2018 103 215.3, the Forward Primer needs to be immobilized on the heating elements. TaqMan-Probe 1 (SEQ ID NO: 13) and TaqMan-Probe 2 (SEQ ID NO: 14) are possible variants for the real-time detection of the amplification reaction.

SIV-2

    Forward Primer: CAGAAGTTATAAGAATGATGGAA (SEQ ID NO: 15)
    Reverse Primer: GCATTGTCTCCGAAGAAATAAGA (SEQ ID NO: 16)
    TaqMan-Probe 1: CGATCGGGTTCGTTGCCTTTTC (SEQ ID NO: 17)
    TaqMan-Probe 2: GAAAAGGCAACGAACCCGATCG (SEQ ID NO: 18)

Either Forward- or Reverse-Primer are immobilized on the heating elements via thiol modification. The respective primer is modified on its 5'-end as follows: 5'thiol - poly-A sequence - Spacer9 - primer-sequence. Hereby, the 5'-thiol moiety functions as covalent coupling agent for coupling the respective primer to the gold surface of the heating elements, the poly-A sequence functions as "spatial buffer" between the surface of the heating elements and primer sequence and can e.g. also improve the steric accessability (being e.g. composed of 5 to 45 adenine bases), whereas Spacer9 is an abasic modification which can prevent the overwriting or complementation of the poly-A sequence through the polymerase.

In case the immobilized primer is also to be used for the extraction of RNA analogously to DE 10 2018 103 215.3, the Forward Primer needs to be immobilized on the heating elements. TaqMan-Probe 1 (SEQ ID NO: 17) and TaqMan-Probe 2 (SEQ ID NO: 18) are possible variants for the real-time detection of the amplification reaction.

**SEQUENCE LISTING**

    SEQ ID NO: 1 ASF-1 Forward Primer
    SEQ ID NO: 2 ASF-1 Reverse Primer
    SEQ ID NO: 3 ASF-1 TaqMan-Probe 1

SEQ ID NO: 4 ASF-1 TaqMan-Probe 2
SEQ ID NO: 5 ASF-1 TaqMan-Probe 3
SEQ ID NO: 6 ASF-1 TaqMan-Probe 4
SEQ ID NO: 7 ASF-2 Forward Primer
SEQ ID NO: 8 ASF-2 Reverse Primer
SEQ ID NO: 9 ASF-2 TaqMan-Probe 1
SEQ ID NO: 10 ASF-2 TaqMan-Probe 2
SEQ ID NO: 11 SIV-1 Forward Primer
SEQ ID NO: 12 SIV-1 Reverse Primer
SEQ ID NO: 13 SIV-1 TaqMan-Probe 1
SEQ ID NO: 14 SIV-1 TaqMan-Probe 2
SEQ ID NO: 15 SIV-2 Forward Primer
SEQ ID NO: 16 SIV-2 Reverse Primer
SEQ ID NO: 17 SIV-2 TaqMan-Probe 1
SEQ ID NO: 18 SIV-2 TaqMan-Probe 2

**REFERENCES**

(1) DE 10 2016 120 124.3
(2) DE 10 2018 103 215.3
(3) DE 19543060
(4) DE 199600398
(5) Duwensee et al., Biosensors and Bioelectronics 2009; 25: 400-405
(6) EP 2 809 806
(7) King et al., J Virol Methods 2003, 107(1), 53-61
(8) Reske et al., Talanta 2007; 74: 393-397
(9) US 7,569,366
(10) US 6,586,233
(11) WO 2007/143034

**Claims**

1.  Diagnostic method for the detection of at least one virus selected from African Swine Fever Virus (ASFV) and/or Swine Influenza Virus (SIV) in at least one ex-vivo sample of one or more animals, comprising the steps:

(a) isolating at least one sample of such one or more animals,
(b) amplifying any present viral genomic nucleic acid contained in such isolated at least one sample by means of a polymerase chain reaction in a reaction volume, wherein the reaction volume is heated through the use of electrical energy, **characterised in that** in at least one of the passages of the amplification cycle of the polymerase chain reaction, the ratio of the electrical energy used in the denaturation step for heating the reaction volume to the size of the reaction volume is less than 20 Joule per millilitre (mL); and
(c) detecting the presence of the amplified viral genomic nucleic acid contained in such isolated at least one sample of such one or more animals.

2. The diagnostic method according to claim 1, wherein a heating means consisting of one or a plurality of electrically contacted heating elements, which are in contact with the reaction volume, heats the reaction volume, **characterised in that** in at least one of the passages of the amplification cycle of the polymerase chain reaction, the heating means supplies to the reaction volume less heat generated in the denaturation step than $C_R * 5°C$, wherein $C_R$ is the heat capacity of the reaction volume during the heating by means of the heating means.

3. The diagnostic method according to claim 2, **characterised in that**, in at least one of the passages of the amplification cycle of the polymerase chain reaction, the maximum increase of the average temperature of the reaction volume, taking place through the denaturation step, is less than 10°C.

4. The diagnostic method according to any one of claims 2 to 3, **characterised in that** in at least one of the passages of the amplification cycle of the polymerase chain reaction, the heating means supplies to the reaction volume less heat generated in the denaturation step than $C_R * 5°C$, wherein $C_R$ is the heat capacity of the reaction volume during the heating by the heating means and, while the heating means is heating the sample, a temporally stable temperature is not established on at least 10% of the contact area of the heating means with the reaction volume.

5. The diagnostic method according to any one of claims 2 to 4, **characterised in that** the ratio between the surface of the heating element(s), which is in contact with the reaction volume, and the reaction volume is greater than 0.1 per meter.

6. The diagnostic method according to any one of claims 2 to 5, **characterised in that** the heat supply through the heating means varies during the polymerase chain reaction.

7. The diagnostic method according to any one of claims 1 to 6, **characterised in that** in at least one of the passages of the amplification cycle of the polymerase chain reaction, the cycle duration $t_c$ is shorter than 60 seconds.

8. The diagnostic method according to any one of claims 1 to 7, **characterised in that** the duration of the polymerase chain reaction $t_{PCR}$ is shorter than 45 minutes.

9. The diagnostic method according to any one of claims 1 to 8, **characterised in that** the polymerase chain reaction according to step (b) is carried out with the nucleic acid sequences (forward and reverse primers) selected from the group consisting of: SEQ ID NOS: 1, 2, 7, 8, 11, 12, 15, 16, preferably SEQ ID NO: 1 + SEQ ID NO: 2, SEQ ID NO: 7 + SEQ ID NO: 8, SEQ ID NO: 11 + SEQ ID NO: 12 or SEQ ID NO: 15 + SEQ ID NO: 16, wherein more preferably the polymerase chain reaction is a solid-phase polymerase chain reaction, wherein even more preferably one of the nucleic acid sequences (forward and reverse primer) selected from the group consisting of: SEQ ID NO: 1 + SEQ ID NO: 2, SEQ ID NO: 7 + SEQ ID NO: 8, SEQ ID NO: 11 + SEQ ID NO: 12 or SEQ ID NO: 15 + SEQ ID NO: 16, is immobilized on the heating elements according to claim 2.

10. The diagnostic method according to claim 9, **characterised in that** such nucleic acid sequences (forward and reverse primers) are directed to ASFV and selected from the group consisting of: SEQ ID NOS: 1, 2, 7, 8.

11. The diagnostic method according to claim 9, **characterised in that** the nucleic acid sequences (forward and reverse primers) are directed to SIV and selected from the group consisting of: SEQ ID NOS: 11, 12, 15, 16.

12. The diagnostic method according to any one of claims 1 to 11, **characterised in that** the detection according to step (c) is carried out with one or more of the nucleic acid sequences (TaqMan probes) selected from the group consisting of: SEQ ID NOS: 3, 4, 5, 6, 9, 10, 13, 14, 17, 18, preferably wherein such nucleic acid sequences (TaqMan probes) are directed to ASFV and selected from the group consisting of: SEQ ID NOS: 3, 4, 5, 6, 9, 10 or wherein such nucleic acid sequences (TaqMan probes) are directed to SIV and selected from the group consisting of: SEQ ID NOS: 13, 14, 17, 18.

13. The diagnostic method according to any one of claims 1 to 12, **characterised in that** the at least one ex-vivo sample is a blood sample or a tissue sample.

14. The diagnostic method according to any one of claims 1 to 13, **characterised in that** the one or more animals is a mammal, preferably swine, more preferably a domestic pig.

15. Use of the amplification method according to any one of claims 1 to 14 for the detection of at least one virus selected from African Swine Fever Virus (ASFV) and/or Swine Influenza Virus (SIV) in at least one ex-vivo sample of one or more animals.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7569366 B
- US 6586233 B
- WO 2007143034 A
- EP 2809806 A
- DE 19543060
- DE 199600398
- WO 2018073435 A
- DE 102013215168
- US 5210015 A
- DE 102016120124
- DE 102018103215

**Non-patent literature cited in the description**

- **RESKE, FLECHSIG et al.** Electrochemical detection of osmium tetroxide- labelled PCR-products by means of protective strands. *Talanta,* 2007, vol. 74, 393-397
- **DUWENSEE, FLECHSIG et al.** Electrochemical product detection of an asymmetric convective polymerase chain reaction. *Biosensors and Bioelectronics,* 2009, vol. 25, 400-405
- **D. RENNEBERG ; C.J. LEUMANN.** Watson-Crick base-pairing properties of Tricyclo-DNA. *J. Am. Chem. Soc.,* 2002, vol. 124, 5993-6002
- **HOLLAND et al.** *Proc Natl Acad Sci US A,* 1991, vol. 88 (16), 7276-7280
- **KING et al.** *J Virol Methods,* 2003, vol. 107 (1), 53-61
- **DUWENSEE et al.** *Biosensors and Bioelectronics,* 2009, vol. 25, 400-405
- **RESKE et al.** *Talanta,* 2007, vol. 74, 393-397